(19) Europäisches Patentamt
European Patent Office
Office européen des brevets

(11) EP 3 353 262 B1

(12) FASCICULE DE BREVET EUROPEEN

(45) Date de publication et mention de la délivrance du brevet:
**25.03.2020 Bulletin 2020/13**

(21) Numéro de dépôt: **16767295.5**

(22) Date de dépôt: **20.09.2016**

(51) Int Cl.:
***C09K 19/32*** *(2006.01)*
***H01M 10/0564*** *(2010.01)*
***H01M 10/052*** *(2010.01)*
***C25B 9/00*** *(2006.01)*
***C07C 309/47*** *(2006.01)*
***H01M 8/1016*** *(2016.01)*
***H01M 8/1018*** *(2016.01)*

(86) Numéro de dépôt international:
**PCT/EP2016/072312**

(87) Numéro de publication internationale:
**WO 2017/050769 (30.03.2017 Gazette 2017/13)**

(54) **ÉLECTROLYTE POUR GÉNÉRATEUR ÉLECTROCHIMIQUE**

ELEKTROLYT FÜR ELEKTROCHEMISCHEN GENERATOR

ELECTROLYTE FOR ELECTROCHEMICAL GENERATOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.09.2015 FR 1558864**

(43) Date de publication de la demande:
**01.08.2018 Bulletin 2018/31**

(73) Titulaire: **Commissariat à l'Énergie Atomique et aux Énergies Alternatives**
**75015 Paris (FR)**

(72) Inventeurs:
- **PICARD, Lionel**
  **38170 Seyssinet-pariset (FR)**
- **GEBEL, Gérard**
  **38120 Saint-Egreve (FR)**
- **LECLERE, Mélody**
  **38110 La-batie-Montgascon (FR)**
- **MENDIL, Hakima**
  **94400 Vitry Sur Seine (FR)**
- **RANNOU, Patrice**
  **38320 Eybens (FR)**

(74) Mandataire: **Nony**
**11 rue Saint-Georges**
**75009 Paris (FR)**

(56) Documents cités:
**EP-A1- 2 792 671**
**EP-A2- 1 176 184**
**JP-A- 2000 336 052**

- **TAKAHIRO ICHIKAWA ET AL: "Induction of Thermotropic Bicontinuous Cubic Phases in Liquid-Crystalline Ammonium and Phosphonium Salts", JOURNAL OF THE AMERICAN CHEMICAL SOCIETY, vol. 134, no. 5, 9 janvier 2012 (2012-01-09), pages 2634-2643, XP055288958, US ISSN: 0002-7863, DOI: 10.1021/ja209010m**
- **TAKAHIRO ICHIKAWA ET AL: "3D Interconnected Ionic Nano-Channels Formed in Polymer Films: Self-Organization and Polymerization of Thermotropic Bicontinuous Cubic Liquid Crystals", JOURNAL OF THE AMERICAN CHEMICAL SOCIETY, vol. 133, no. 7, 27 janvier 2011 (2011-01-27), pages 2163-2169, XP055288963, US ISSN: 0002-7863, DOI: 10.1021/ja106707z**
- **JUNJI SAKUDA ET AL: "Liquid-Crystalline Electrolytes for Lithium-Ion Batteries: Ordered Assemblies of a Mesogen-Containing Carbonate and a Lithium Salt", ADVANCED FUNCTIONAL MATERIALS, WILEY-VCH, WEINHEIM, vol. 25, no. 8, 25 février 2015 (2015-02-25), pages 1206-1212, XP001595313, DOI: 10.1002/ADFM.201402509**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux composés pouvant être mis en œuvre comme électrolytes, notamment dans des systèmes de génération ou de stockage électrochimique.

De tels électrolytes peuvent être utilisés dans différents systèmes ou dispositifs électrochimiques, notamment dans des batteries au lithium.

D'une manière classique, le principe de fonctionnement d'un générateur électrochimique repose sur l'insertion et le retrait, aussi appelé « désinsertion », d'un ion de métal alcalin ou d'un proton, dans et depuis l'électrode positive, et le dépôt ou l'extraction de cet ion, sur et de l'électrode négative.

Les principaux systèmes utilisent le cation lithium comme espèce ionique de transport. Dans le cas d'un accumulateur au lithium par exemple, le cation lithium extrait de la cathode lors de la charge de la batterie vient se déposer sur l'anode, et inversement, il s'extrait de l'anode pour s'intercaler dans la cathode lors de la décharge.

Le transport du proton ou du cation alcalin ou alcalino-terreux, en particulier du cation lithium, entre la cathode et l'anode, est assuré par un électrolyte conducteur ionique.

La formulation de l'électrolyte utilisé revêt un caractère essentiel pour les performances du système électrochimique, en particulier lorsque celui-ci est utilisé à des températures très basses ou très élevées. La conductivité ionique de l'électrolyte conditionne notamment l'efficacité du système électrochimique étant donné qu'elle intervient sur la mobilité des ions entre les électrodes positive et négative.

D'autres paramètres interviennent également dans le choix de l'électrolyte mis en œuvre. Il s'agit notamment de sa stabilité thermique, chimique ou électrochimique au sein du système électrochimique, ainsi que des critères économiques, de sécurité et de respect de l'environnement, incluant notamment la toxicité de l'électrolyte.

D'une manière générale, les électrolytes des systèmes électrochimiques se présentent sous forme liquide, gélifiée ou solide.

En ce qui concerne les électrolytes sous forme liquide, les électrolytes conventionnels de générateurs électrochimiques avec un cation métallique de l'une des deux premières colonnes du tableau périodique des éléments, par exemple au lithium, sont composés d'un sel de ce cation dissous dans un milieu organique ou aqueux (classiquement dans des solvants carbonates, acétonitrile pour les batteries au lithium), en présence ou non d'additifs.

Par exemple, les électrolytes conventionnels de supercapacité sont composés d'un sel organique (classiquement un sel de tétraéthylammonium tétrafluoroborate $Et_4N$-$BF_4$) dissous dans de l'acétonitrile.

Leur utilisation en système complet de stockage électrochimique, par exemple dans une batterie Li-ion, nécessite toutefois d'ajouter un séparateur pour assurer une isolation électrique entre les électrodes positive et négative. Egalement, même si ces électrolytes présentent de bonnes conductivités ioniques, ils posent toutefois des problèmes de sécurité et de coût dans le cadre de la mise en œuvrede solvants organiques (stabilité thermique faible), et de stabilité électrochimique dans le cadre de la mise en œuvre d'un milieu aqueux.

Concernant les électrolytes gélifiés, il s'agit d'électrolytes liquides, par exemple tels que décrits précédemment, emprisonnés dans un polymère « hôte ». Le ou les solvants de l'électrolyte liquide doivent présenter une affinité avec le polymère hôte, ni trop élevée (solubilisation du polymère), ni trop faible (exsudation). Le polymère hôte doit permettre une incorporation maximale de liquide tout en conservant des propriétés mécaniques pour garantir la séparation physique entre les deux électrodes.

Enfin, pour répondre aux problématiques de sécurité liées à la présence du solvant, il a été proposé de mettre en œuvre des électrolytes polymères solides. Ces polymères entrant dans la composition de l'électrolyte doivent présenter de bonnes propriétés de conduction ionique afin de pouvoir être utilisés de façon satisfaisante dans des systèmes générateurs et de stockage électrochimiques.

Il est par exemple connu d'utiliser comme électrolytes polymères ne nécessitant pas l'utilisation d'un séparateur, des poly(oxyéthylène) POE dans lesquels est dissous un sel de métal alcalin ou alcalino-terreux (suivant la chimie des électrodes). Toutefois, ces électrolytes présentent des performances limitées en matière de conductivité ionique liées au mécanisme de transport du cation, dit « assisté », et nécessitent une température d'utilisation élevée (60 °C à 80 °C). Les polymères sont ainsi conducteurs dans un état physique gélifié.

On peut également citer comme électrolyte polymère, la membrane électrolytique des systèmes de générateur électrochimique de type pile à combustible à membrane d'échange de protons, classiquement constituée d'un polymère à chaîne principale carbofluorée et porteur de groupements pendants comportant des fonctions acide sulfonique, tel que le Nafion®. A l'heure actuelle, l'utilisation de ce type de polymères pour la conduction protonique nécessite toutefois de maitriser le taux d'hydratation de la membrane pour obtenir les performances souhaitées. Ce type de polymère est un polymère semi-cristallin, dont seule la partie amorphe présente des propriétés de conduction, la partie cristalline conférant les propriétés mécaniques nécessaires à son bon fonctionnement en système complet.

Différentes études ont été menées en vue d'augmenter les performances de conduction ionique des électrolytes polymères.

Par exemple, la demande internationale WO 00/05774 décrit des copolymères à blocs à micro-séparation de

phases, constitués d'un premier bloc conducteur ionique, par exemple d'oxyde de polyéthylène, et d'un second bloc, non conducteur et non miscible avec le premier bloc pour assurer une micro-séparation de phase, par exemple de type polyalkylacrylate ou polydiméthylsiloxane. Ces électrolytes polymères ne nécessitent pas l'ajout d'un sel additionnel puisqu'un anion (par exemple carboxylate, sulfonate ou phosphate) est immobilisé sur le polymère.

Il a également été proposé un mélange d'un polystyrène porteur de groupements sulfonyl(trifluorométhylsulfonyl)imide et de POE pour réaliser une membrane d'électrolyte (Meziane et al. Electrochimica Acta, 2011, 57, 14-19). Ces électrolytes polymères présentent néanmoins des conductivités ioniques insuffisantes, de l'ordre de $9{,}5.10^{-6}$ S/cm à 70 °C. De plus, il n'est pas possible, pour la plupart des domaines d'application actuels, de mettre en œuvre des températures de fonctionnement supérieures à 70 °C.

Enfin, on peut encore citer le document FR 2 979 630 qui propose un électrolyte solide de type copolymère di-bloc de type BA ou tri-bloc de type BAB, avec A une chaîne polyoxyéthylène non substituée et B un polymère anionique formé à partir d'un ou plusieurs monomères de type vinylique et dérivés, substitués par un anion sulfonyl(trifluorométhylsulfonyl)imide (TFSI). La conductivité maximale, de l'ordre de $10^{-5}$ S/cm, est obtenue à 60 °C avec un polymère comportant 78 % en masse de POE.

La publication Ichikawa et al., Journal of the American Chemical Society, vol. 134, no. 5, pages 2634-2643, propose la mise en œuvre de composés cristal liquide thermotrope en combinaison avec un sel de lithium pour obtenir des films polymériques conducteurs d'ions lithium.

Le document JP 2000 336052 décrit la mise en œuvre d'une phase cristal liquide discotique formée par des composés présentant un squelette polyaromatique, pour créer des structures pour le transport d'ions, pouvant être mises en œuvre conjointement à un sel de lithium pour former un électrolyte.

Quant au document EP 2 792 671, il propose également des composés de type cristal liquide et leur mise en œuvre en combinaison avec un sel de lithium pour former un électrolyte

Pour des raisons évidentes, l'amélioration des performances des électrolytes relève d'un objectif permanent.

Il demeure donc un besoin de disposer d'un électrolyte possédant une conductivité ionique élevée et possédant de préférence un nombre de transport le plus proche de l'unité possible.

Il demeure également un besoin de disposer d'un électrolyte possédant une stabilité électrochimique améliorée, notamment sur une plage de température élargie.

La présente invention vise précisément à proposer de nouveaux électrolytes, conducteurs ioniques, cationiques ou protoniques, présentant une conductivité ionique et une stabilité électrochimique améliorées.

Plus particulièrement, l'invention décrit une molécule cristal liquide ionique thermotrope comportant :

- une partie, dite rigide, constituée d'un groupement polycyclique Ar formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
- une partie, dite flexible, formée d'une ou plusieurs chaînes aliphatiques, linéaires ou ramifiées, saturées ou insaturées, fluorées ou non, la ou lesdites chaînes étant éventuellement interrompues par un ou plusieurs hétéroatomes, par un ou plusieurs métalloïdes et/ou par un ou plusieurs (hétéro)cycles, aromatiques ou non, de 4 à 6 chaînons, et éventuellement substituées par un ou plusieurs groupes choisis dans le groupe constitué des groupes hydroxyle, $-NH_2$ et oxo ;
  ladite partie flexible étant liée de manière covalente, directement ou *via* un espaceur, à ladite partie rigide ; et
- un ou plusieurs groupements ioniques $-A^{x-}C^{x+}$,

  $-A^{x-}$ étant un groupement anionique lié de manière covalente à ladite partie rigide, avec x entier égal à 1 ou 2, $-A^{x-}$ étant choisi dans le groupe constitué des anions sulfonate, sulfonylimide de formule $-SO_2-N^--SO_2C_yF_{2y+1}$ avec y entier variant de 0 à 4, borate, borane, phosphate, phosphinate, phosphonate, silicate, carbonate, sulfure, sélénate, nitrate et perchlorate ; et
  $C^{x+}$ étant un contre-cation du groupement anionique $-A^{x-}$, choisi dans le groupe constitué de $H^+$ et des cations des métaux alcalins et alcalino-terreux.

L'invention a plus particulièrement pour objet, selon un premier de ses aspects, une molécule cristal liquide ionique thermotrope telle que définie en revendication 1.

Un cristal liquide thermotrope se définit par trois types d'états successifs, que celui-ci revêt en fonction de la température.

En deçà de sa température de fusion, il est dans un état cristallin (ou phase cristalline). Puis, au delà de sa température de fusion, il passe dans un état mésomorphe constitué d'une mésophase ou d'une succession de mésophases. Enfin, au delà de sa température de clarification, il passe dans un état isotrope (ou phase amorphe).

Par « température de fusion », on entend la température à laquelle un cristal liquide thermotrope passe d'un état cristallin à un état mésomorphe.

Par « température de clarification », on entend la température à laquelle un cristal liquide thermotrope quitte sa mésophase ou sa dernière mésophase d'une succession de mésophases pour entrer dans un état isotrope (ou liquide).

Par « état mésomorphe », on entend l'état dans lequel se trouve un cristal liquide thermotrope lorsqu'il est porté à une température supérieure à sa température de fusion et inférieure à sa température de clarification.

Par « cristal liquide ionique », on entend un cristal liquide portant au moins un groupement ionique, appelé $-A^{x-}C^{x+}$.

Comme illustré dans les exemples qui suivent, les inventeurs ont montré que, lorsque les molécules cristal liquide ionique thermotrope de l'invention sont dans un état mésomorphe, celles-ci présentent une conductivité ionique (cationique ou anionique) ou protonique.

La plage de température dans laquelle une molécule cristal liquide thermotrope est dans un état mésomorphe peut être déterminée par une méthode connue de l'homme du métier, comme par exemple la DSC (« Differential Scanning Calorimetry » ou calorimétrie différentielle à balayage).

Cette méthode de caractérisation permet également de mesure les températures de fusion et de clarification.

La nature des mésophases d'un état mésomorphe peut être déterminée par une combinaison d'autres caractérisations tel que par MOP (Microscope Optique en lumière Polarisée), par DRX (Diffraction des Rayons X) et/ou par SAXS (« Small Angle X-ray Scattering » ou diffusion des rayons X aux petits angles), cette dernière étant généralement utilisé en complément de la DRX.

Ainsi, l'invention concerne, selon un autre de ses aspects, l'utilisation d'une molécule cristal liquide ionique thermotrope telle que définie en revendication 1, dans un état mésomorphe, à titre d'électrolyte dans un système électrochimique.

L'invention concerne encore un électrolyte comprenant, voire étant formé, de molécules cristal liquide ionique thermotrope telles que définies en revendication 1, dans un état mésomorphe.

Les molécules selon l'invention peuvent être mises en œuvre comme électrolytes dans de nombreux systèmes électrochimiques, tels que les générateurs, par exemple les batteries au lithium, et les systèmes de conversion électrochimique, par exemple les piles à combustible à membrane échangeuse de protons (PEMFC).

La mise en œuvre des molécules selon l'invention comme électrolytes s'avère avantageuse à plusieurs titres.

Tout d'abord, ces molécules étant conductrices ioniques dans un état mésomorphe, elles présentent une température d'utilisation comme électrolyte fortement élargie, pouvant être dans toute la gamme de température dans laquelle les molécules sont dans un état mésomorphe, qui correspond généralement à la gamme de température entre la température de fusion et la température de clarification. Les molécules de l'invention peuvent en outre être conductrices ioniques à une température située au-delà de leur température de clarification.

Un système électrochimique, par exemple une batterie au lithium, réalisé à partir d'un électrolyte selon l'invention, peut ainsi fonctionner sur une large plage de température, de préférence entre -60 °C et +300 °C, et plus préférentiellement entre -20 °C et +200 °C.

Par ailleurs, la conductivité ionique d'un électrolyte selon l'invention est basée sur un mécanisme de conduction « direct », par « saut » (« hopping » en langue anglaise) des cations $C^{x+}$ d'un groupement anionique-$A^{x-}$, situé sur un groupement polycyclique Ar, à l'autre, et non sur un mécanisme assisté comme c'est le cas par exemple des électrolytes polymères proposés par Cohen et al. Molecular Transport in Liquids and Glasses, J. Chem. Phys. 31, 1164 (1959). Sans vouloir être lié à une théorie particulière, ce mécanisme est permis par l'arrangement des molécules de l'invention en un état mésomorphe.

Un électrolyte selon l'invention conduit ainsi à des performances améliorées en termes de conductivité ionique, protonique ou cationique.

D'autres caractéristiques, variantes et avantages des molécules et électrolytes selon l'invention, de leur préparation et de leur mise en œuvre ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

## MOLECULES DE L'INVENTION

Dans le cadre de l'invention, on entend par :

- « alkyle », un groupe aliphatique saturé, linéaire ou ramifié ; par exemple un groupe $C_{1-4}$-alkyle représente une chaîne carbonée de 1 à 4 atomes de carbone, linéaire ou ramifiée, plus particulièrement un méthyle, éthyle, propyle, isopropyle, butyle, isobutyle, *tert*-butyle ;
- « (hétéro)cycle aromatique ou non de 4 à 6 chaînons », un groupe cyclique insaturé, partiellement saturé ou saturé,

à 4, 5 ou 6 chaînons, comprenant éventuellement un ou plusieurs hétéroatomes, en particulier choisis dans le groupe constitué de l'oxygène, le soufre et l'azote. Un cycle aromatique peut être notamment le benzène ;

- « groupement polycyclique », un groupement présentant deux ou plusieurs noyaux (cycles), condensés (ortho-condensés ou ortho- et péri-condensés) les uns aux autres, c'est-à-dire présentant, deux à deux, au moins deux carbones en commun.

Le groupement polycyclique peut inclure un ou plusieurs hétéroatomes. On parle alors de « groupement polyhétérocyclique ».

- « métaux alcalins », les éléments chimiques de la première colonne du tableau périodique des éléments, et plus particulièrement choisis dans le groupe constitué du lithium, sodium, potassium, rubidium, et césium. De préférence, le métal alcalin est le lithium, le sodium ou le potassium, et plus préférentiellement le lithium ;
- « métaux alcalino-terreux », les éléments chimiques de la deuxième colonne du tableau périodique des éléments, et plus particulièrement choisis dans le groupe constitué du béryllium, magnésium, calcium, strontium, baryum, et radium. De préférence, le métal alcalino-terreux est le magnésium ou le potassium ;
- « métalloïdes », les éléments chimiques suivants ; bore, silicium, germanium, arsenic, antimoine, tellure, et astate. De préférence, le métalloïde est le bore ou le silicium ;
- « espaceur », un atome ou un groupe d'atomes de valence au moins égale à 2, reliant de manière covalente la partie rigide et la partie flexible. Dans le cadre de l'invention revendiquée, l'espaceur relie la partie rigide à deux chaînes aliphatiques de la partie flexible et l'espaceur est un atome ou un groupe d'atomes trivalent. A titre d'espaceur adapté, on peut citer les atomes d'azote, et de phosphore.

*Partie rigide*

Ar est un groupement polycyclique comprenant de 2 à 4 cycles ayant de 4 à 6 chaînons, de au moins l'un des cycles étant aromatique et représentant de préférence un cycle benzénique.

Selon l'invention, Ar est un groupement polycyclique aromatique formé de 2 à 4 cycles benzéniques fusionnés.

Plus particulièrement, et toujours selon l'invention, Ar présente l'un des squelettes polycycliques suivants :

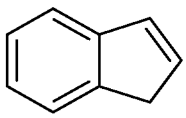
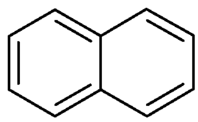
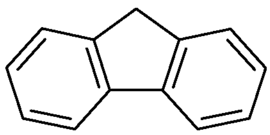
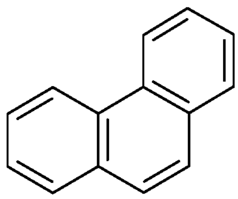
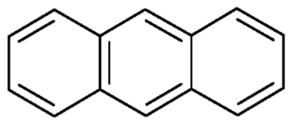
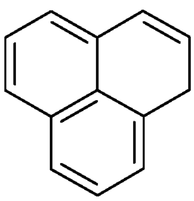
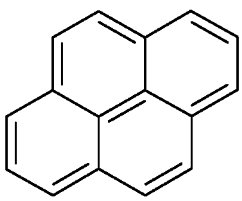
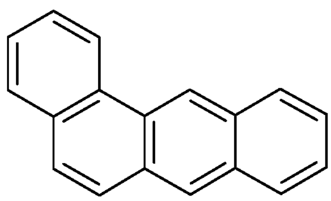
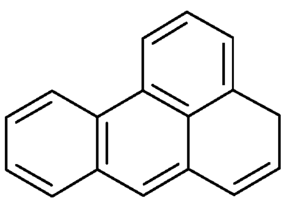
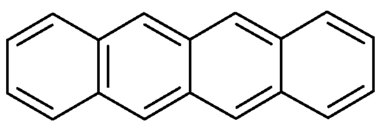

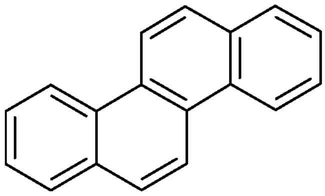

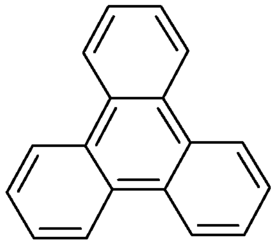

Il est entendu que le groupement Ar peut être un groupement polyhétérocyclique présentant l'un des squelettes présentés ci-dessus dans lequel un ou plusieurs atomes de carbone sont remplacés par un ou plusieurs hétéroatomes, notamment choisis dans le groupe constitué de S, N et O.

Selon un mode de réalisation particulier, Ar est un groupement bicyclique aromatique, en particulier présentant un squelette aromatique naphtalène.

De préférence, Ar est un groupe naphtalène.

*Partie flexible*

De préférence, la ou les chaînes aliphatiques de la partie flexible comprennent de 4 à 18 atomes de carbone.

De préférence, la ou les chaînes aliphatiques de la partie flexible est (sont) substituée(s) par au moins un, voire un, groupe hydroxyle.

Selon un mode de réalisation, la partie flexible est formée de deux chaînes aliphatiques, linéaires ou ramifiées, chacune des chaînes présentant un enchaînement linéaire d'au moins 6 liaisons covalentes.

Selon un mode de réalisation particulier, chacune des chaînes aliphatiques est formée d'un unique segment ou d'un enchaînement linéaire d'au moins deux segments de chaîne, en particulier de deux ou trois segments de chaîne de nature chimique différente.

De préférence, chacune des chaînes aliphatiques représente une chaîne alkyle linéaire comprenant de 6 à 18 atomes de carbone, de préférence de 6 à 12 atomes de carbone, éventuellement fluorée, éventuellement substituée par au moins un, voire un, groupe hydroxyle, et éventuellement entrecoupée par un ou plusieurs atomes d'oxygène.

Selon l'invention, la partie flexible est formée de deux chaînes aliphatiques, et celles-ci sont liées de manière covalente *via* un unique espaceur trivalent au même atome de carbone du groupement Ar formant la partie rigide.

Ledit atome de valence égale à 3, en particulier ledit atome d'azote, fait office d'espaceur reliant de manière covalente lesdites chaînes aliphatiques formant ladite partie flexible et le groupement Ar formant la partie rigide.

De préférence, les deux chaînes aliphatiques sont liées de manière covalente au même atome de carbone du groupement Ar formant la partie rigide *via* un unique atome d'azote.

Selon un mode de réalisation particulier, la partie flexible est formée de deux chaînes alkyles linéaires, identiques ou différentes, de préférence identiques, comprenant de 4 à 18 atomes de carbone, de préférence de 6 à 16 atomes de carbone, éventuellement substituées par un ou plusieurs groupes hydroxyle, éventuellement entrecoupées par un ou plusieurs atomes d'oxygène, lesdites chaînes étant liées au même atome de carbone du groupement Ar *via* un unique atome d'azote.

Selon un mode de réalisation préféré, la partie flexible est formée de deux chaînes alkyles linéaires, comprenant de 6 à 16 atomes de carbone, chacune étant substituée par un groupe hydroxyle et éventuellement entrecoupée par un atome d'oxygène, lesdites chaînes étant liées au même atome de carbone du groupement Ar *via* un unique atome d'azote.

*Groupement ionique*

Dans les molécules cristal liquide ionique thermotrope de l'invention, le groupement anionique $-A^{x-}$ peut être plus particulièrement choisi dans le groupe constitué des anions sulfonate (de formule $-SO_3^-$) et des anions de formule $-SO_2-N^--SO_2-C_yF_{2y+1}$ avec y variant de 0 à 4, de préférence égal à 1.

De préférence, $-A^{x-}$ est un anion sulfonate.

De préférence, $-A^{x-}$ est un anion de formule $-SO_2-N^--SO_2-CF_3$.

Selon une variante de réalisation de l'invention, $C^{x+}$ représente le proton $H^+$.

Selon cette variante, le groupement $-A^{x-}C^{x+}$ représente de préférence un groupe $-SO_3^-H^+$, où l'atome de soufre est lié de manière covalente à un atome de carbone ou un hétéroatome de la partie rigide.

Comme détaillé dans la suite du texte, de telles molécules peuvent être avantageusement mises en œuvre à titre d'électrolyte dans une pile à combustible à membrane échangeuse de protons (PEMFC) ou un électrolyseur basse température.

Selon une autre variante de réalisation de l'invention, $C^{x+}$ représente le cation $Li^+$.

Selon cette variante, le groupement $-A^{x-}C^{x+}$ représente de préférence un groupe $-SO_3^-Li^+$, où l'atome de soufre est lié de manière covalente à un atome de carbone ou un hétéroatome de la partie rigide.

Comme détaillé dans la suite du texte, de telles molécules peuvent être avantageusement mises en œuvre à titre d'électrolyte dans une batterie au lithium.

Selon un mode de réalisation particulier, les molécules selon l'invention présentent la structure suivante :

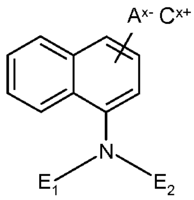
(I)

dans laquelle $-A^{x-}$ et $C^{x+}$ sont tels que définis ci-dessus, et $E_1$ et $E_2$ représentent des chaînes aliphatiques, identiques ou différentes, telles que définies ci-dessus.

De préférence, $E_1$ et $E_2$ représentent des chaînes aliphatiques identiques.

De préférence, la formule générale (I) répond à l'une des deux sous-formules ci-dessous :

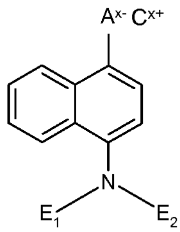
(I-1) ou

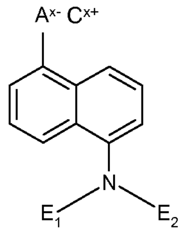
(I-2).

dans lesquelles $-A^{x-}$ et $C^{x+}$ sont tels que définis ci-dessus, et $E_1$ et $E_2$ représentent des chaînes aliphatiques, identiques ou différentes, telles que définies ci-dessus.

Selon un mode de réalisation particulier, les chaînes $E_1$ et $E_2$ représentent des chaînes alkyles linéaires, identiques ou différentes, comprenant de 4 à 18 atomes de carbone, de préférence de 6 à 16 atomes de carbone, éventuellement fluorées, éventuellement substituées par un ou plusieurs groupes hydroxyle, éventuellement entrecoupées par un ou plusieurs atomes d'oxygène.

Selon un mode de réalisation préféré, les chaînes $E_1$ et $E_2$ représentent des chaînes alkyles linéaires, identiques ou différentes, comprenant de 6 à 16 atomes de carbone, chacune étant substituée par un groupe hydroxyle, éventuellement fluorées, et éventuellement entrecoupées par un atome d'oxygène.

Les chaînes $E_1$ et $E_2$ répondent de préférence à la formule générale $-CH_2-CHOH-R$, où R est un groupe alkyle en $C_4$-$C_{18}$, éventuellement fluoré, éventuellement entrecoupé d'un ou plusieurs atomes d'oxygène.

De préférence, les molécules cristal liquide ionique thermotrope conformes à l'invention ne sont pas des polymères.

Avantageusement, les molécules cristal liquide ionique thermotrope conformes à l'invention présentent une masse moléculaire inférieure ou égale à 1 500 g/mol, préférentiellement inférieure à 1 000 g/mol.

Les molécules cristal liquide ionique thermotrope conformes à l'invention sont particulièrement efficaces à titre d'électrolyte lorsque le rapport massique partie flexible/partie rigide (excluant le ou les groupements ioniques) est compris de 1/1 à 100/1, préférentiellement de 1/1 à 50/1, avantageusement de 1/1 à 10/1.

La présente invention a notamment pour objet les molécules cristal liquide ionique thermotrope suivantes, particulièrement appropriées à une utilisation comme électrolyte :

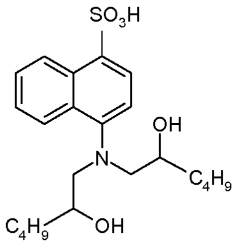
6-ANH

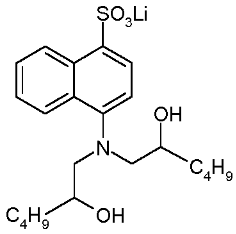
6-ANLi

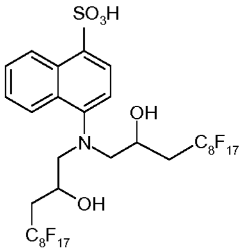
8F-ANH

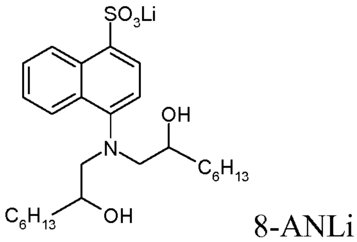
8-ANLi

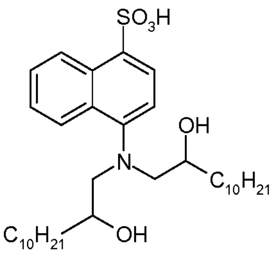
12-ANH

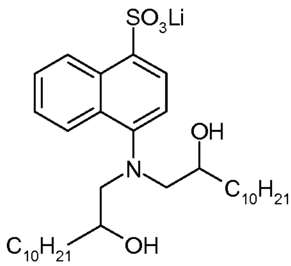
12-

ANLi

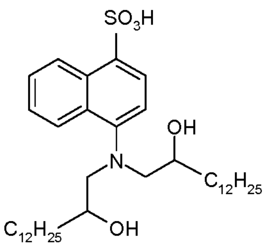
14-ANH

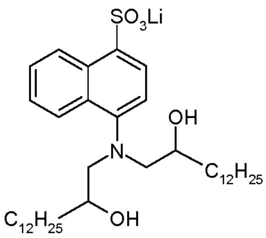
14-ANLi

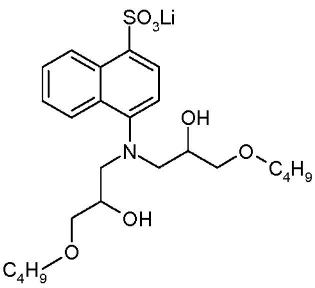
BGE-ANLi

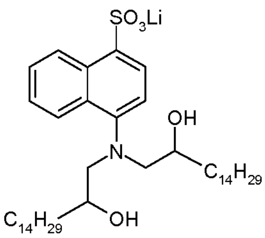
16-ANLi

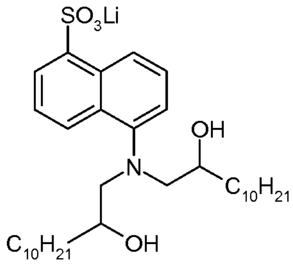

12-AN'Li

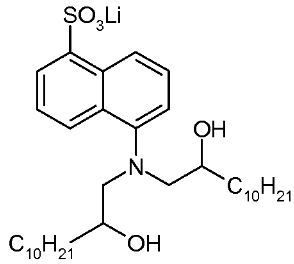

16-AN'Li

*Préparation des composés de l'invention*

Les molécules selon l'invention peuvent être préparées en mettant en œuvre des méthodes de substitution ou d'addition nucléophile connues de l'homme du métier, comme détaillé ci-dessous.

Les molécules de l'invention peuvent être préparées en mettant en présence, dans des conditions propices à leur interaction selon une réaction de substitution ou d'addition nucléophile connues de l'homme du métier, un précurseur de la partie rigide et un précurseur de la partie flexible.

Le précurseur de la partie rigide est avantageusement porteur du ou des groupements ioniques $-A^{x-}c^{x+}$ ou alternativement porteur d'un ou plusieurs précurseurs desdits groupements ioniques.

Selon un mode de réalisation, le précurseur de la partie rigide est porteur d'un groupe nucléophile, par exemple de type amine, hydroxyle ou sulfure, et le précurseur de la partie flexible est porteur d'un groupe électrophile de type époxyde, halogène, isocyanate, nitrile, thiocarbonyle ou carbonyle.

Alternativement, le précurseur de la partie flexible est porteur d'un groupe nucléophile, par exemple de type amine, hydroxyle ou sulfure, et le précurseur de la partie rigide est porteur d'un groupe électrophile de type époxyde, halogène, isocyanate, nitrile, thiocarbonyle ou carbonyle.

À titre illustratif des méthodes que l'homme du métier peut employer pour parvenir aux molécules de l'invention, une méthode de préparation des molécules de l'invention de formule (I) est décrite ci-après et également dans les exemples qui suivent.

Les molécules de l'invention de formule (I) peuvent être préparées selon un procédé comprenant au moins la mise en présence d'un composé présentant la formule (I') suivante :

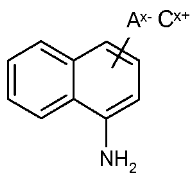

(I')

avec un précurseur de la chaîne $E_1$ et un précurseur de la chaîne $E_2$, lesdits précurseurs étant éventuellement identiques lorsque $E_1$ et $E_2$ sont identiques, dans des conditions propices à leur interaction selon une réaction d'addition nucléophile.

Cette réaction est de préférence réalisée dans un solvant aprotique polaire, tel que le diméthylformamide.

Cette réaction est de préférence réalisée en chauffant le mélange réactionnel composé du composé de formule (I'), des précurseurs et dudit solvant, à une température comprise de 80 °C à 120 °C.

Cette réaction est de préférence réalisée en présence d'au moins un équivalent du précurseur de la chaîne $E_1$ et d'au moins un équivalent du précurseur de la chaîne $E_2$, par rapport au composé de formule (I').

Les précurseurs des chaînes $E_1$ et $E_2$ sont avantageusement porteurs d'un groupe électrophile de type époxyde, halogène, isocyanate, nitrile, thiocarbonyle ou carbonyle, de préférence d'un groupe époxyde.

Les molécules de l'invention de formule (I) où $E_1$ et $E_2$ sont identiques peuvent être obtenues *via* une réaction d'addition nucléophile entre un composé de formule (I') et un précurseur des chaînes $E_1$ et $E_2$, porteur d'un groupe électrophile, comme par exemple un groupe époxyde. Cette réaction est de préférence réalisée en présence d'au moins deux équivalents du précurseur des chaînes $E_1$ et $E_2$ par rapport au composé de formule (I').

Bien entendu, il appartient à l'homme du métier d'ajuster les conditions de synthèse pour obtenir les molécules selon l'invention.

**Utilisation comme électrolyte**

Les molécules cristal liquide ionique thermotrope selon l'invention peuvent avantageusement être utilisées,

dans un état mésomorphe, à titre d'électrolyte dans un système électrochimique.

Comme mentionné ci-dessus, un état mésomorphe désigne la mésophase ou la succession de mésophases dans laquelle les molécules cristal liquide ionique thermotrope selon l'invention se trouvent en fonction de leur température, située entre la température de fusion et la température de clarification.

L'électrolyte formé de telles molécules est avantageusement mis en œuvre en association avec un séparateur poreux sur lequel ledit électrolyte est imprégné, ledit séparateur assurant une séparation physique entre les deux électrodes du système électrochimique.

A titre de séparateur, on peut utiliser tout séparateur poreux conventionnellement utilisé dans un système électrochimique, comme par exemple un séparateur poreux de batterie au lithium ou une membrane échangeuse d'ions d'une pile à combustible. L'homme du métier est en mesure de choisir un séparateur adapté à la mise en œuvre de l'électrolyte.

Les molécules cristal liquide ionique thermotrope selon l'invention dans lesquelles $C^{x+}$ représente un cation $Li^+$ peuvent avantageusement être utilisées, dans un état mésomorphe, à titre d'électrolyte dans une batterie au lithium.

Les molécules cristal liquide ionique thermotrope selon l'invention dans lesquelles $C^{x+}$ représente $H^+$ peuvent avantageusement être utilisées, dans un état mésomorphe, à titre d'électrolyte dans une pile à combustible à membrane échangeuse de protons, ou un électrolyseur basse température.

## Electrolyte

Comme évoqué précédemment, les molécules cristal liquide ionique thermotrope selon l'invention sont conductrices ioniques, protoniques ou cationiques, dans leur état mésomorphe.

La présente invention se rapporte à un électrolyte comprenant, voire étant formé, de molécules cristal liquide ionique thermotrope telles que définies ci-dessus, dans un état mésomorphe.

Dans l'électrolyte de l'invention, les molécules cristal liquide ionique thermotrope sont de préférence mises en œuvre à une température comprise de 80 °C à 220 °C, généralement comprise de 100 °C à 200 °C, préférentiellement comprise de 130 °C à 170 °C, par exemple de l'ordre de 150 °C.

De préférence, l'électrolyte liquide de l'invention présente une viscosité supérieure ou égale à 10 mPa.s, de préférence comprise de 100 mPa.s à 100 Pa.s, à une température comprise entre -60 °C et 300 °C.

Par « à une température comprise entre -60 °C et 300 °C », on entend que l'électrolyte liquide de l'invention présente une viscosité telle que définie ci-dessus à au moins une température située dans cette plage. Ceci ne signifie pas nécessairement que l'électrolyte liquide de l'invention présente une viscosité telle que définie ci-dessus à toute température située dans cette plage.

La viscosité peut être mesurée par extrapolation à cisaillement nul de la courbe de viscosité en fonction du gradient de cisaillement à une température donnée, mesurée sur un viscosimètre/rhéomètre cône/plan ou plan/plan.

Cette condition sur la viscosité de l'électrolyte liquide assure une bonne imprégnation de celui-ci dans le séparateur du système électrochimique.

L'électrolyte selon l'invention présente de bonnes propriétés de conductivité ionique.

De préférence, l'électrolyte de l'invention présente une conductivité ionique à 20 °C supérieure ou égale à $10^{-9}$ S/cm, en particulier comprise entre $10^{-7}$ S/cm et $10^{-5}$ S/cm et une conductivité ionique à 200 °C supérieure ou égale à $10^{-5}$ S/cm.

La conductivité ionique peut être mesurée par spectroscopie d'impédance électrochimique en tension ou en courant, d'après une méthode connue de l'homme du métier.

## Système électrochimique

L'électrolyte selon l'invention peut être mis en œuvre dans un système électrochimique, par exemple pour une batterie au lithium.

La présente invention concerne ainsi, selon encore un autre de ses aspects, un système électrochimique comprenant un électrolyte selon l'invention.

Dans le système électrochimique de l'invention, l'électrolyte est de préférence imprégné sur un séparateur poreux tel que décrit précédemment.

Le système électrochimique peut être un système générateur, convertisseur ou de stockage électrochimique.

Il peut s'agir plus particulièrement d'une pile à combustible, par exemple une pile à combustible à membrane échangeuse de protons (PEMFC) ; une batterie primaire ou secondaire, par exemple une batterie au lithium, sodium, magnésium, potassium ou calcium ; un accumulateur lithium-air ou lithium-soufre.

Selon un mode de réalisation particulier, l'électrolyte est mis en œuvre dans une batterie, en particulier une batterie au lithium.

La présente invention concerne également, selon encore un autre de ses aspects, un séparateur poreux

imprégné d'un électrolyte selon l'invention.

Un tel séparateur poreux est particulièrement adapté à sa mise en œuvre dans un système électrochimique tel que décrit ci-dessus.

L'invention va maintenant être décrite au moyen des exemples et figures suivants, donnés bien entendu à titre illustratif et non limitatif de l'invention.

Figure 1 : Analyse calorimétrique du produit 12-ANH par DSC sous argon et avec une vitesse de chauffage de 10°K/min.
Figure 2 : Analyse du produit 12-ANH par DRX.
Figure 3 : Isothermes de sorption/désorption d'eau à 25 °C du produit 12-ANH.
Figure 4 : Analyse du produit 12-ANH par SAXS, en fonction de son taux d'hydratation.
Figure 5 : Analyse calorimétrique du produit 12-ANLi par DSC sous argon et avec une vitesse de chauffage de 10°K/min.
Figure 6 : Analyse du produit 12-ANLi par DRX.
Figure 7 : Isothermes de sorption/désorption d'eau à 25 °C du produit 12-ANLi.
Figure 8 : Analyse de conductivité ionique, en montée et en descente de température, des produits 12-ANLi et 14-ANLi.
Figure 9 : Analyse de conductivité ionique, en montée de température, des produits 12-ANLi, 14-ANLi, 16-ANLi, 12-AN'Li et 16-AN'Li.

## EXEMPLES

### *Préparation d'intermédiaires de synthèse*

Préparation de 4-amino-1-naphtalènesulfonate de lithium (ANLi)

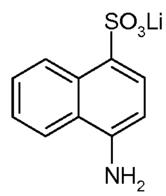

1,340 g d'acide 4-amino-1-naphtalènesulfonique (ANH) et 0,210 g d'hydroxyde de lithium monohydrate (LiOH, $H_2O$) ont été ajoutés dans 20 mL d'eau distillée. Le milieu réactionnel a été agité pendant 1 nuit à température ambiante. L'excès d'ANH a été éliminé par filtration. Le filtrat a ensuite été concentré par évaporation du solvant sous pression réduite. Après deux lavages à l'éthanol, on a obtenu une poudre rose (ANLi).
RMN $^1$H (400 MHz ; DMSO-d6 ; 300 K) : $\delta$ ppm 7,89 (dd, 1H); 7,18 (dd, 1H); 6,83 (d, 1H); 6,52 (m, 2H); 5,68 (d, 1H); 4,96 (s, 2H).
RMN $^{13}$C (400 MHz; DMSO-d6 ; 300 K) : $\delta$ ppm 146,11 ; 132,34; 130,61 ; 128,10 ; 126,45 ; 125,33 ; 123,56 ; 122,94 ; 122,26 ; 105,17.

Préparation de 5-amino-1-naphtalènesulfonate de lithium (AN'Li)

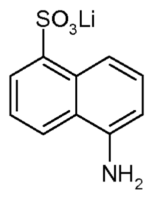

24,441 g d'acide 4-amino-1-naphtalènesulfonique (ANH) et 4,282 g d'hydroxyde de lithium monohydrate (LiOH,

$H_2O$) ont été ajoutés dans 500 mL d'eau distillée. Le milieu réactionnel a été agité pendant 1 nuit à température ambiante. L'excès d'ANH a été éliminé par filtration. Le filtrat a ensuite été concentré par évaporation du solvant sous pression réduite. Après deux lavages à l'éthanol, on a obtenu une poudre rose (AN'Li).

***Exemple 1 - Préparation d'un cristal liquide 12-ANH conforme à l'invention***

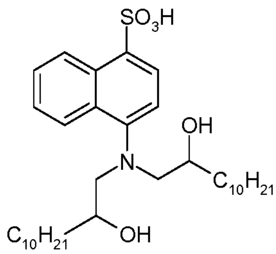

2,0 g (8,96 mmol) d'acide 4-amino-1-naphtalènesulfonique (ANH) et 3,29 g (17,9 mmol) de 1,2-époxydodécane (soit 2 équivalents d'époxyde pour une amine) dans 10 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé à la température de 80 °C en début de réaction puis ré-élevé à 100 °C pendant 2 jours jusqu'à obtention d'un mélange bi-phasique marron. Les produits de la réaction ont été précipités dans un large volume de diéthyl éther, puis filtrés sur Büchner. La phase solide a ensuite été dissoute dans le méthanol, ce dernier étant retiré dans un évaporateur rotatif.
Le produit obtenu (12-ANH) a été séché sous pression réduite et une pâte marron a été obtenue.

Caractérisation du cristal liquide 12-ANH

- Le produit 12-ANH a été caractérisé par DSC sous argon et avec une vitesse de chauffage de 10°K/min. Les résultats de l'analyse calorimétrique sont représentés en figure 1.
  Le spectre DSC montre la transition de phase à -38 °C (correspondant à la température de fusion) et un pic endothermique à 193 °C (correspondant à la température de clarification).
- Le cristal liquide 12-ANH a été observé en MOP. Le cristal liquide est placé entre deux plaques de verres hydrophiles (épaisseur du dépôt : 3-5 $\mu$m), glissées à l'intérieur d'une plaque chauffante et sous atmosphère contrôlée (azote), elle-même montée entre le polariseur et l'analyseur du microscope.
  Le cliché MOP obtenu après cisaillement sous les plaques de verre à 185 °C montre l'apparition de très petite zone biréfringentes représentatives de l'observation des défauts de phases mésomorphes.
- Un dépôt de poudre de 12-ANH a été réalisé sur un support en verre pour analyse DRX.
  Le diffractogramme des rayons X, représenté en figure 2, montre que le cristal liquide 12-ANH présente une phase lamellaire hexagonale par des pics de diffraction dans des rapports $\sqrt{3},\ \sqrt{4},\ \sqrt{7}$.
- L'évaluation de l'hygroscopie du cristal liquide 12-ANH a été réalisée en conduisant l'étude de l'isotherme de sorption/désorption d'eau en phase vapeur conformément à al méthode décrite ci-après.

La balance de sorption a été équipée d'une microbalance électronique et d'un analyseur de point de rosée. Le cristal liquide 12-ANH a été préalablement séché à 60 °C dans une étuve tirée sous vide. Puis le cristal liquide 12-ANH a été à nouveau séché dans la balance à 60 °C avec une rampe de 5 °C/min jusqu'à atteindre un équilibre de 0,0010 % de prise en masse pendant 10 minutes. Si ces conditions ne sont pas satisfaites, le cristal liquide a été séché jusqu'à une durée maximale de 600 minutes. La condition de fin utilisée était 0,005 % en changement de masse pour une durée de 20 minutes. Durant le cycle de sorption/désorption, l'attente maximale pour atteindre l'équilibre a été de 1000 minutes.
La figure 3 représente les isothermes de sorption/désorption d'eau à 25 °C, montrant que le cristal liquide 12-ANH est hydrophile.

- Le cristal liquide 12-ANH a également été caractérisé par SAXS, en fonction de son taux d'hydratation.

Le taux d'hydratation des échantillons a été contrôlé à partir d'une atmosphère contrôlée. Le cristal liquide 12-ANH est séché à l'étuve à 60°C pendant une semaine sous vide pour obtenir un échantillon sec. Le cristal liquide 12-ANH sec a été ensuite analysé par SAXS et l'humidité relative de la pièce a été mesurée à l'aide d'un hygromètre. On a laissé ensuite l'échantillon s'équilibrer pendant 1, 3, et 6 heures. Pour obtenir un échantillon hydraté dans une atmosphère à 100 % d'humidité, on l'a placé dans une coupelle pour éviter le contact direct avec l'eau. Cette même coupelle a été placée dans un système hermétique rempli d'eau. On a alors obtenu un système saturé en eau où l'échantillon peut s'hydrater.

On observe un déplacement du « pic ionomère » avec l'hydratation du cristal liquide 12-ANH (figure 4).

Le « pic ionomère » est la signature de la séparation de phase entre domaine hydrophile et hydrophobe à l'échelle nanométrique. Sa position (q) traduit directement l'état de gonflement nanométrique ou la distance de corrélation entre les domaines hydrophiles (ou hydrophobes) ($d=2\ \pi/q$). Plus ce pic est décalé vers les petites valeurs de « q » (plus d augmente), plus la quantité d'eau dans le produit est importante.

***Exemple 2 - Préparation d'un cristal liquide 12-ANLi conforme à l'invention***

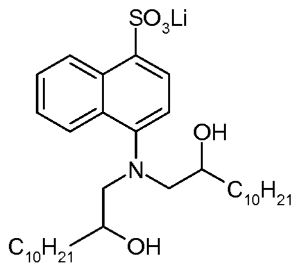

Ce produit a été synthétisé selon un protocole similaire à celui décrit en exemple 1, en utilisant 1,050 g d'ANLi (4,73 mmol) au lieu de l'ANH avec 1,741 g (9,46 mmol) de 1,2-époxydodécane dans 10 mL de DMF. La réaction est maintenue à 70 °C pendant 3 jours. On obtient une poudre jaune marron (12-ANLi).

Caractérisation du cristal liquide 12-ANLi

- Les résultats de l'analyse DSC sous argon et avec une vitesse de chauffage de 10°K/min sont représentés en figure 5. Le spectre DSC montre la transition de phase à 6,25 °C (correspondant à la température de fusion) et un pic endothermique à 226 °C (correspondant à la température de clarification).
- Un dépôt de poudre de 12-ANLi a été réalisé sur un support en verre pour analyse par diffraction des rayons X.

L'analyse par DRX représenté en figure 6 montre que le composé 12-ANLi présente une phase lamellaire par des pics de diffraction dans des rapports 2, 3, 4, 5.

- L'évaluation de l'hygroscopie du cristal liquide 12-ANLi a été réalisée en conduisant l'étude de l'isotherme de sorption/désorption d'eau en phase vapeur, selon le protocole détaillé en exemple 1.

La figure 7 représente les isothermes de sorption/désorption d'eau à 25 °C, montrant que le cristal liquide 12-ANLi est hydrophile.

***Exemple 3 - Préparation du cristal liquide 6-ANH conforme à l'invention***

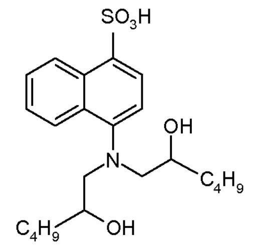

3,101 g (13,47 mmol) de ANH et 3,35 mL (26,94 mmol) de 1,2-époxyhexane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 100°C pendant 120 h jusqu'à obtention d'un mélange homogène marron. Les produits de la réaction ont été précipités dans un large volume de diéthyl éther, puis filtrés sur Büchner.

Le produit obtenu (6-ANH) a été séché sous pression réduite et une pâte marron a été obtenue.

***Exemple 4 - Préparation du cristal liquide 6-ANLi conforme à l'invention***

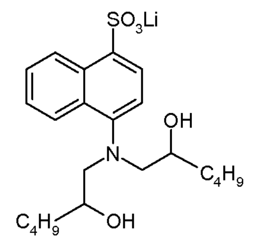

3,122 g (13,62 mmol) de ANLi et 3,39 mL (27,24 mmol) de 1,2-époxyhexane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 100°C pendant 144 h jusqu'à obtention d'un mélange homogène rouge très foncé. Les produits de la réaction ont été précipités dans un large volume de diéthyl éther, puis filtrés sur Büchner.

Le produit obtenu (6-ANLi) a été séché sous pression réduite et une pâte rouge lie-de-vin a été obtenue.

Les observations par SAXS en température ont confirmé l'existence d'une structure organisée, prouvant que 6-ANLi est un cristal liquide ionique thermotrope.

***Exemple 5 - Préparation du cristal liquide 8-ANLi conforme à l'invention***

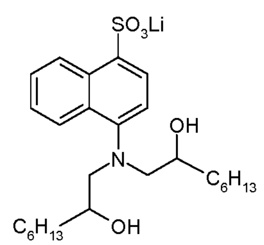

3,03 g (13,19 mmol) de ANLi et 4,21 mL (26,38 mmol) de 1,2-époxyoctane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 60°C pendant 96 h, puis à 65 °C pendant 144 h, puis à 75 °C pendant 264 h, puis à 85 °C pendant 3 semaines jusqu'à obtention d'un mélange homogène très sombre. Les produits de la réaction ont été précipités dans un large volume de diéthyl éther, puis filtrés sur Büchner.

Le produit obtenu (8-ANLi) a été séché sous pression réduite et une poudre rouge foncé a été obtenue.

***Exemple 6 - Préparation du cristal liquide 14-ANH conforme à l'invention***

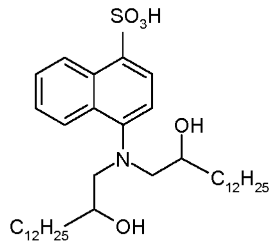

3,136 g (13,62 mmol) de ANH et 8,057 mL (27,25 mmol) de 1,2-époxytétradécane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 90°C pendant 144 h jusqu'à obtention d'un mélange homogène rouge très foncé. Les produits de la réaction ont été précipités dans un large volume de diéthyl éther, puis filtrés sur Büchner.
Le produit obtenu (14-ANH) a été séché sous pression réduite et une poudre rouge orange a été obtenue.

***Exemple 7 - Préparation du cristal liquide 14-ANLi conforme à l'invention***

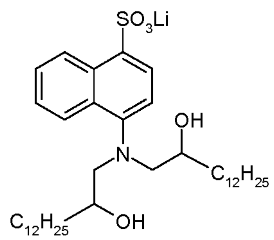

3,008 g (13,12 mmol) de ANLi et 7,76 mL (26,25 mmol) de 1,2-époxytétradécane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 90°C pendant 144 h jusqu'à obtention d'un mélange homogène marron très foncé. Les produits de la réaction ont été précipités dans un large volume de diéthyl éther, puis filtrés sur Büchner.
Le produit obtenu (14-ANLi) a été séché sous pression réduite et une poudre rouge a été obtenue.
Les observations par DSC, MOP et SAXS ont confirmé que 14-ANLi est un cristal liquide ionique thermotrope.
Le spectre DSC ne pas montre la transition de phase correspondant à la température de fusion (car inférieure à la température minimale de la mesure) mais présente trois pics endothermiques caractéristiques de trois changements de phase mésomorphe.
L'évolutions des spectres SAXS en montée et descente en température, ainsi que les clichés MOP, ont permis d'identifier les trois phase mésomorphes : lamellaire, lamello-colonnaire et colonnaire.

***Exemple 8 - Préparation du cristal liquide 16-ANLi conforme à l'invention***

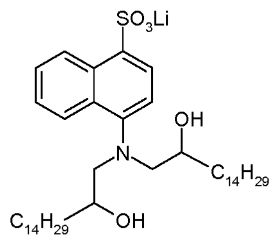

2,991 g (13,12 mmol) de ANLi et 7,380 g (26,10 mmol) de 1,2-époxyhexadécane dans 25 mL de diméthylformamide DMF ont été placés dans un ballon de 100 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 100°C pendant 2 semaines jusqu'à obtention d'un mélange homogène marron très foncé. Les produits de la réaction ont été précipités dans un large volume de diéthyl éther, puis filtrés sur Büchner.

Le produit obtenu (16-ANLi) a été séché sous pression réduite et une poudre rouge a été obtenue.

Les observations par DSC et SAXS ont confirmé que 16-ANLi est un cristal liquide ionique thermotrope présentant deux phases mésomorphes principales de type lamellaire avec une transition de phase mésomorphe autour de 50 °C.

***Exemple 9 - Préparation du cristal liquide 8F-ANH conforme à l'invention***

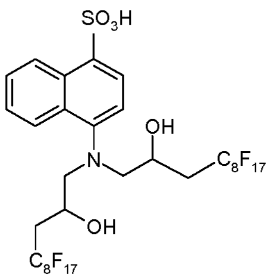

1,00 g d'ANH (4,48 mmol) et 4,26 g (8,95 mmol) de 1,2-époxy-1H,1H,2H,3H,3H-heptadécafluorodécane dans 10 mL de DMF ont été placés dans un ballon équipé d'un réfrigérant et d'un barreau aimanté. Le mélange réactionnel a été maintenu sous agitation et chauffé à 80 °C sous air pendant 2 jours jusqu'à obtention d'un mélange homogène. Le composé a été précipité dans le diéthyl éther et filtré sur Büchner. Le produit a été séché sous pression réduite, et un gel rosâtre stable a été obtenu (8F-ANH).

***Exemple 10 - Préparation du cristal liquide BGE-ANLi conforme à l'invention***

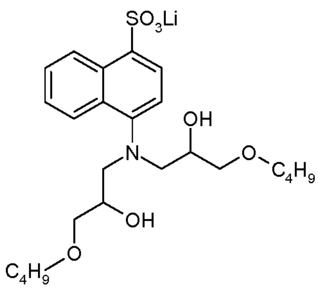

3,034 g (13,24 mmol) de ANLi et 3,99 mL (26,48 mmol) de butyl glycidyléther ont été placés dans un ballon de 25 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 70 °C pendant 24 h, puis à 80 °C pendant 96 h, puis à 90 °C pendant une semaine jusqu'à obtention d'un mélange homogène très sombre. Les produits de la réaction ont été précipités dans un large volume de diéthyl éther, puis filtrés sur Büchner.

Le produit obtenu (BGE-ANLi) a été séché sous pression réduite et une poudre rouge a été obtenue.

***Exemple 11 - Préparation du cristal liquide 12-AN'Li conforme à l'invention***

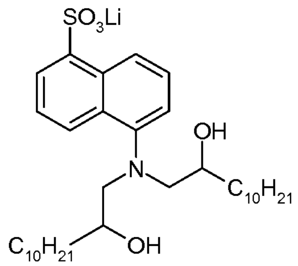

2,903 g (12,67 mmol) de AN'Li et 6,15 mL g (25,33 mmol) de 1,2-époxydodécane dans 15 mL de diméthylformamide DMF ont été placés dans un ballon de 50 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 100 °C pendant 2 semaines jusqu'à obtention d'un mélange homogène marron très foncé. Les produits de la réaction ont été précipités dans un large volume de diéthyl éther, puis filtrés sur Büchner.
Le produit obtenu (12-AN'Li) a été séché sous pression réduite et une poudre rouge a été obtenue.
Les observations par DSC et SAXS ont confirmé que 12-AN'Li est un cristal liquide ionique thermotrope présentant une organisation indéfinie entre 10 °C et 70 °C, puis de type lamellaire au delà de 70 °C.

***Exemple 12 - Préparation du cristal liquide 16-AN'Li conforme à l'invention***

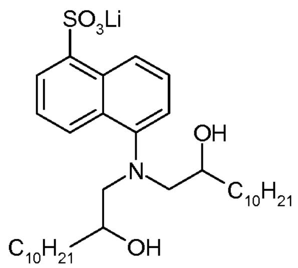

3,160 g (13,79 mmol) de AN'Li et 7,80 g (27,57 mmol) de 1,2-époxyhexadécane dans 25 mL de diméthylformamide DMF ont été placés dans un ballon de 100 mL monté sur un montage à reflux équipé d'un réfrigérant et d'un barreau aimanté, ainsi qu'un bain d'huile sur plaque chauffante. Le mélange réactionnel a été maintenu sous agitation et chauffé sous argon à la température de 90 °C pendant 2 semaines jusqu'à obtention d'un mélange homogène marron très foncé. Les produits de la réaction ont été précipités dans un large volume de diéthyl éther, puis filtrés sur Büchner.
Le produit obtenu (16-AN'Li) a été séché sous pression réduite et une poudre rouge a été obtenue.
Les observations par DSC et SAXS ont confirmé que 16-AN'Li est un cristal liquide ionique thermotrope présentant une organisation colonnaire de la température ambiante à 80 °C, puis une phase lamellaire jusqu'à 160 °C (température de clarification).

***Exemple 13 - Mesures de conductivité ionique***

Les mesures ont été réalisées entre le plan supérieur et inférieur d'un rhéomètre plan/plan utilisant des géométries jetables dans un four permettant d'avoir des mesures de conductivité jusqu'à 250 °C. Les deux plans parallèles représentent les deux électrodes de mesures de spectroscopie d'impédance électrochimique et elles ont été connectées à l'électrode de travail et aux contre-électrodes d'un spectromètre d'impédance.
Le produit à tester a été placé sur la géométrie inférieure et la géométrie supérieure est descendue jusqu'à une force appliquée de 5 N. Le système a été chauffé jusqu'à obtenir la fusion du produit tout en maintenant une force de 5 N, afin d'assurer une surface de contact parfaite entre l'échantillon et les électrodes. Au fur et à mesure que la température a augmenté, l'entrefer de mesure a diminué et la valeur (affichée de façon digitale sur le rhéomètre) a été relevée afin de pouvoir calculer la conductivité ionique. Une spectroscopie d'impédance est réalisée avec une amplitude de 10 mV de 1 MHz à 10 mHz pour chaque température.
Les résultats obtenus sont indiqués dans les Figures 8 et 9 (conductivité ionique en S/cm en fonction de 1000/T, où T est la température en kelvin).
Les molécules cristal liquide ionique thermotrope conformes à l'invention 12-ANLi, 12-AN'Li, 14-ANLi, 16-ANLi

et 16-AN'Li présentent une conductivité ionique sur une large plage de température, comprise d'environ $10^{-9}$ S/cm à environ 60 °C à environ $10^{-5}$ S/cm à environ 200 °C.

Ces molécules cristal liquide ionique thermotrope conformes à l'invention sont appropriées à une utilisation en tant qu'électrolyte dans un système électrochimique, en particulier dans une batterie au lithium.

## Revendications

**1.** Molécule cristal liquide ionique thermotrope, **caractérisée en ce qu'**elle comporte :

- une partie, dite rigide, constituée d'un groupement polycyclique Ar formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ; ledit groupement polycyclique Ar présentant l'un des squelettes suivants :

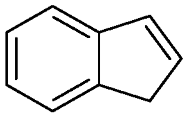
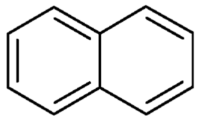
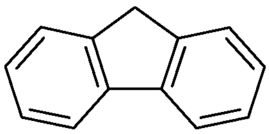
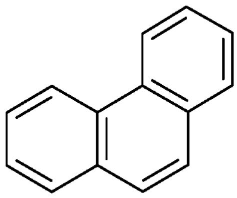
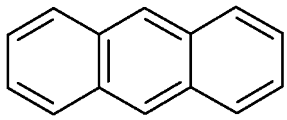
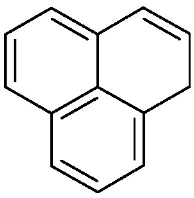
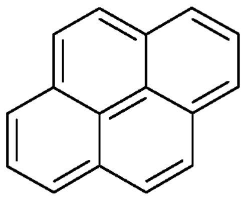
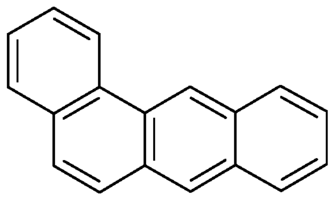
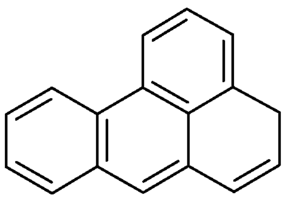
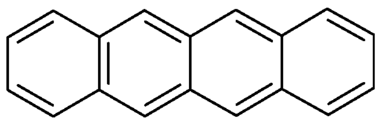
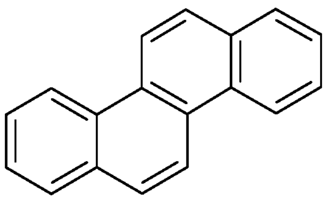
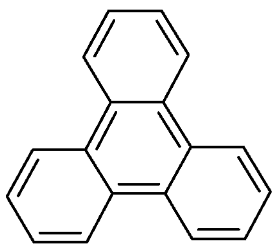

- une partie, dite flexible, formée de deux chaînes aliphatiques, linéaires ou ramifiées, saturées ou insaturées, fluorées ou non, la ou lesdites chaînes étant éventuellement interrompues par un ou plusieurs hétéroatomes, par un ou plusieurs métalloïdes et/ou par un ou plusieurs (hétéro)cycles, aromatiques ou non, de 4 à 6 chaînons, et éventuellement substituées par un ou plusieurs groupes choisis dans le groupe constitué des groupes hydroxyle, $-NH_2$ et oxo ;

lesdites chaînes aliphatiques étant liées de manière covalente *via* un unique espaceur trivalent à un même atome de carbone du groupement Ar formant la partie rigide ; ledit espaceur étant un atome ou un groupes d'atomes de valence égale à 3 ; et

• un ou plusieurs groupements ioniques $-A^{x-}C^{x+}$,

$-A^{x-}$ étant un groupement anionique lié de manière covalente à ladite partie rigide, avec x entier égal à 1 ou 2, $-A^{x-}$ étant choisi dans le groupe constitué des anions sulfonate, sulfonylimide de formule $-SO_2-N^--SO_2C_yF_{2y+1}$ avec y entier variant de 0 à 4, borate, borane, phosphate, phosphinate, phosphonate, silicate, carbonate, sulfure, sélénate, nitrate et perchlorate ; et

$C^{x+}$ étant un contre-cation du groupement anionique $-A^{x-}$, choisi dans le groupe constitué de $H^+$ et des cations des métaux alcalins et alcalino-terreux.

2. Molécule selon la revendication 1, dans laquelle ledit groupement polycyclique Ar est un groupement bicyclique aromatique, en particulier présentant un squelette aromatique naphtalène, et plus particulièrement Ar est un groupe naphtalène.

3. Molécule selon l'une quelconque des revendications précédentes, dans laquelle la partie flexible est formée : de deux chaînes aliphatiques, linéaires ou ramifiées, chacune des chaînes présentant un enchaînement linéaire d'au moins 6 liaisons covalentes.

4. Molécule selon l'une quelconque des revendications précédentes, dans laquelle chacune des chaînes aliphatiques est formée d'un unique segment ou d'un enchaînement linéaire d'au moins deux segments de chaîne, en particulier de deux ou trois segments de chaîne de nature chimique différente.

5. Molécule selon l'une quelconque des revendications précédentes, dans laquelle ladite partie flexible est formée de deux chaînes alkyles linéaires, comprenant de 4 à 18 atomes de carbone, éventuellement substituées par un ou plusieurs groupes hydroxyle, éventuellement entrecoupées par un ou plusieurs atomes d'oxygène.

6. Molécule selon l'une quelconque des revendications précédentes, dans laquelle le ou lesdits groupements anioniques $-A^{x-}$ sont des anions sulfonate ou et des anions de formule $-SO_2-N^--SO_2-C_yF_{2y+1}$ avec y variant de 0 à 4 et de préférence égal à 1, en particulier des anions sulfonate.

7. Molécule selon l'une quelconque des revendications précédentes, dans laquelle $C^{x+}$ est $H^+$ ou le cation $Li^+$.

8. Molécule selon l'une quelconque des revendications précédentes, **caractérisée en ce qu'**elle présente la structure suivante :

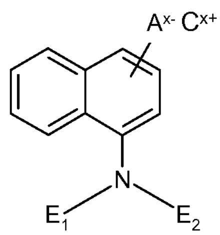

(I)

dans laquelle $A^{x-}$ et $C^{x+}$ sont tels que définis selon l'une quelconque des revendications 1, 6 et 7, et $E_1$ et $E_2$ représentent des chaînes aliphatiques, identiques ou différentes, telles que définies selon l'une quelconque des revendications 1 et 3 à 5, en particulier les chaînes $E_1$ et $E_2$ représentent des chaînes alkyles linéaires, identiques ou différentes, comprenant de 6 à 16 atomes de carbone, chacune étant substituée par un groupement hydroxyle, éventuellement fluorées, et éventuellement entrecoupées par un atome d'oxygène.

9. Utilisation de molécules cristal liquide ionique thermotrope telles que définies selon l'une quelconque des revendications 1 à 8, dans un état mésomorphe, à titre d'électrolyte dans un système électrochimique.

10. Utilisation selon la revendication précédente, dans laquelle $C^{x+}$ représente un cation $Li^+$, à titre d'électrolyte dans une batterie au lithium.

**11.** Utilisation selon la revendication 10, dans laquelle $C^{x+}$ représente $H^+$, à titre d'électrolyte dans une pile à combustible à membrane échangeuse de protons, ou un électrolyseur basse température.

**12.** Electrolyte comprenant, voire étant formé, de molécules cristal liquide ionique thermotrope telles que définies selon l'une quelconque des revendications 1 à 8, dans un état mésomorphe, ledit électrolyte présentant en particulier viscosité supérieure ou égale à 10 mPa.s à une température comprise entre -60 °C et 300°C.

**13.** Electrolyte selon la revendication 12, **caractérisé en ce qu'**il présente une conductivité ionique à 20 °C supérieure ou égale à $10^{-9}$ S/cm, en particulier comprise entre $10^{-7}$ S/cm et $10^{-5}$ S/cm et une conductivité ionique à 200 °C supérieure ou égale à $10^{-5}$ S/cm.

**14.** Système électrochimique comprenant un électrolyte tel que défini selon l'une quelconque des revendications 12 et 13, ledit électrolyte étant de préférence imprégné sur un séparateur poreux, ledit système électrochimique étant plus particulièrement une batterie, en particulier une batterie au lithium.

**15.** Séparateur poreux **caractérisé en ce qu'**il est imprégné d'un électrolyte tel que défini selon l'une quelconque des revendications 12 et 13.

## Patentansprüche

**1.** Thermotropes ionisches Flüssigkristallmolekül, **dadurch gekennzeichnet, dass** es Folgendes umfasst:

• einen als starr bezeichneten Teil, bestehend aus einer polycyclischen Gruppe Ar aus zwei bis sechs Ringen, von denen mindestens eine aromatisch ist, wobei die Ringe unabhängig voneinander 4 bis 6 Glieder umfassen, wobei die polycyclische Gruppe bis zu 18 Heteroatome, insbesondere aus der Gruppe bestehend aus S, N und O, enthalten kann; wobei die polycyclische Gruppe Ar eines der folgenden Gerüste aufweist:

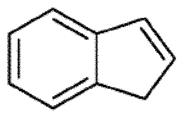
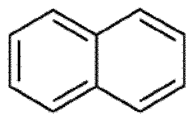
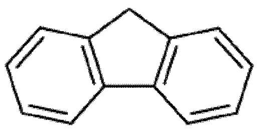
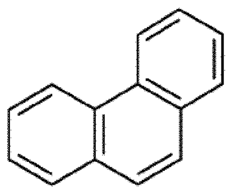
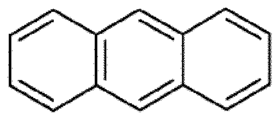
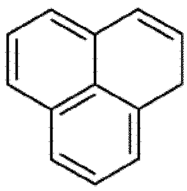
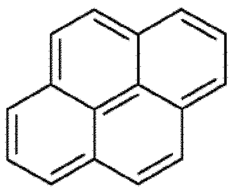
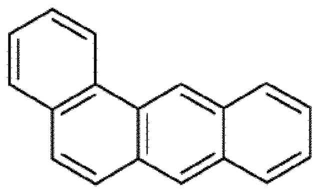
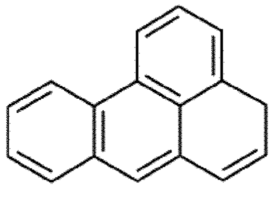
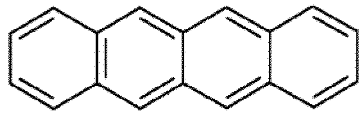

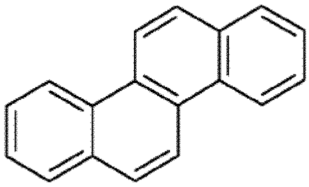

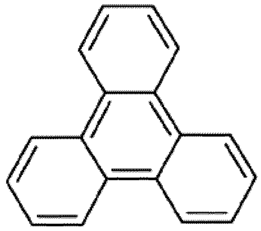

• einen als flexibel bezeichneten Teil aus zwei linearen oder verzweigten, gesättigten oder ungesättigten, fluorierten oder nichtfluorierten aliphatischen Ketten, wobei die Kette bzw. die Ketten gegebenenfalls durch ein oder mehrere Heteroatome, durch ein oder mehrere Metalloide und/oder durch einen oder mehrere aromatische oder nichtaromatische (Hetero)cyclen mit 4 bis 6 Gliedern unterbrochen und gegebenenfalls durch eine oder mehrere Gruppen, die aus der Gruppe bestehend aus Hydroxyl-, $-NH_2$- und Oxogruppen ausgewählt sind, substituiert ist bzw. sind;
wobei die aliphatischen Ketten über einen einzigen dreiwertigen Spacer mit demselben Kohlenstoffatom der Gruppe Ar, die den starren Teil bildet, gebunden sind; wobei es sich bei dem Spacer um einen Atom oder eine Gruppe von Atomen mit einer Wertigkeit von 3 handelt; und
• eine oder mehrere ionische Gruppen $-A^{x-}C^{x+}$,
wobei $-A^{x-}$ für eine anionische Gruppe steht, die kovalent an den starren Teil gebunden ist, wobei x für eine ganze Zahl mit einem Wert von 1 oder 2 steht, wobei $-A^{x-}$ aus der Gruppe bestehend aus Sulfonat-Anionen, Sulfonylimid-Anionen der Formel $-SO_2-N^--SO_2C_yF_{2y+1}$, wobei y für eine ganze Zahl im Bereich von 0 bis 4 steht, Borat-, Boran-, Phosphat-, Phosphinat-, Phosphonat-, Silikat-, Carbonat-, Sulfid-, Selenat-, Nitrat- und Perchlorat-Anionen ausgewählt ist und
wobei $C^{x+}$ für ein Gegenkation der anionischen Gruppe $-A^{x-}$ steht, das aus der Gruppe bestehend aus $H^+$ und Alkali- und Erdalkalimetallkationen ausgewählt ist.

2. Molekül nach Anspruch 1, wobei es sich bei der polycyclischen Gruppe Ar um eine aromatische bicyclische Gruppe, die insbesondere ein aromatisches Naphthalingerüst aufweist, handelt und es sich bei Ar spezieller um eine Naphthalingruppe handelt.

3. Molekül nach einem der vorhergehenden Ansprüche, wobei der flexible Teil aus zwei linearen oder verzweigten aliphatischen Ketten gebildet ist, wobei jede der Ketten eine lineare Sequenz von mindestens 6 kovalenten Bindungen aufweist.

4. Molekül nach einem der vorhergehenden Ansprüche, wobei jede der aliphatischen Ketten aus einem einzigen Segment oder einer linearen Sequenz von mindestens zwei Kettensegmenten, insbesondere aus zwei oder drei chemisch verschiedenartigen Kettensegmenten gebildet ist.

5. Molekül nach einem der vorhergehenden Ansprüche, wobei der flexible Teil aus zwei linearen Alkylketten mit 4 bis 18 Kohlenstoffatomen, die gegebenenfalls durch eine oder mehrere Hydroxylgruppen substituiert und gegebenenfalls durch ein oder mehrere Sauerstoffatome unterbrochen sind, gebildet ist.

6. Molekül nach einem der vorhergehenden Ansprüche, wobei es sich bei der anionischen Gruppierung bzw. den anionischen Gruppierungen $-A^{x-}$ um Sulfonat-Anionen oder Anionen der Formel $-SO_2-N^--SO_2C_yF_{2y+1}$, wobei y für eine ganze Zahl im Bereich von 0 bis 4 steht und vorzugsweise gleich 1 ist, insbesondere Sulfonat-Anionen, handelt.

7. Molekül nach einem der vorhergehenden Ansprüche, wobei $C^{x+}$ für $H^+$ oder das $Li^+$-Kation steht.

8. Molekül nach einem der vorhergehenden Ansprüche, **dadurch gekennzeichnet, dass** es die folgende Struktur aufweist:

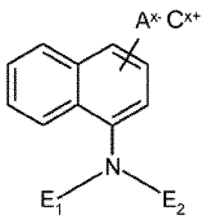

(I)

wobei $A^{x-}$ und $C^{x+}$ wie gemäß einem der Ansprüche 1, 6 und 7 definiert sind und $E_1$ und $E_2$ für gleiche oder verschiedene aliphatische Ketten gemäß der in einem der Ansprüche 1 und 3 bis 5 angegebenen Definition stehen, insbesondere die Ketten $E_1$ und $E_2$ für gleiche oder verschiedene lineare Alkylketten mit 6 bis 16 Kohlenstoffatomen, die jeweils durch eine Hydroxylgruppe substituiert, gegebenenfalls fluoriert und gegebenenfalls durch ein Sauerstoffatom unterbrochen sind, stehen.

**9.** Verwendung von thermotropen ionischen Flüssigkristallmolekülen gemäß einem der Ansprüche 1 bis 8 in mesomorphem Zustand als Elektrolyt in einem elektrochemischen System.

**10.** Verwendung nach dem vorhergehenden Anspruch, wobei $C^{x+}$ für ein $Li^+$-Kation steht, als Elektrolyt in einer Lithiumbatterie.

**11.** Verwendung nach Anspruch 10, wobei $C^{x+}$ für $H^+$ steht, als Elektrolyt in einer Protonenaustauschmembran-Brennstoffzelle oder einem bei niedriger Temperatur arbeitenden Elektrolyseur.

**12.** Elektrolyt, der thermotrope ionische Flüssigkristallmoleküle gemäß einem der Ansprüche 1 bis 8 in mesomorphem Zustand umfasst oder sogar daraus gebildet ist, wobei der Elektrolyt insbesondere eine Viskosität größer oder gleich 10 mPa.s bei einer Temperatur zwischen -60 °C und 300 °C aufweist.

**13.** Elektrolyt nach Anspruch 12, **dadurch gekennzeichnet, dass** er eine Ionenleitfähigkeit bei 20 °C größer oder gleich $10^{-9}$ S/cm, insbesondere zwischen $10^{-7}$ S/cm und $10^{-5}$ S/cm und eine Ionenleitfähigkeit bei 200 °C größer oder gleich $10^{-5}$ S/cm aufweist.

**14.** Elektrochemisches System, umfassend einen Elektrolyt gemäß einem der Ansprüche 12 und 13, wobei der Elektrolyt vorzugsweise auf einem porösen Separator imprägniert ist, wobei es sich bei dem elektrochemischen System spezieller um eine Batterie, insbesondere eine Lithiumbatterie, handelt.

**15.** Poröser Separator, **dadurch gekennzeichnet, dass** er mit einem Elektrolyt gemäß einem der Ansprüche 12 und 13 imprägniert ist.

## Claims

**1.** Thermotropic ionic liquid crystal molecule, **characterized in that** it comprises:

• a "rigid" part, constituted of a polycyclic group Ar formed from two to six rings, at least one of which is aromatic, said rings being, independently of each other, 4-to 6-membered, said polycyclic group possibly including up to 18 heteroatoms, chosen in particular from the group constituted of S, N and O; said polycyclic group Ar having one of the following backbones:

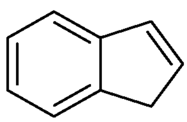

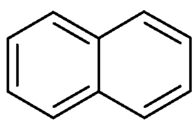

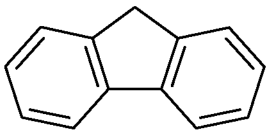 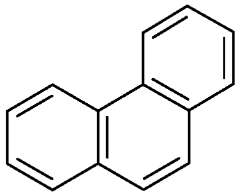 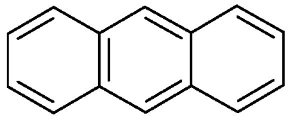

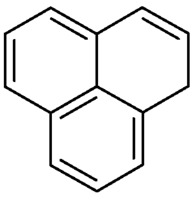 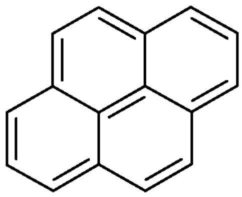 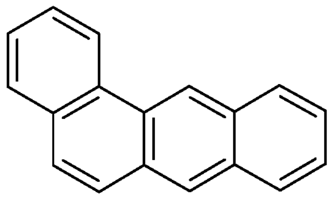

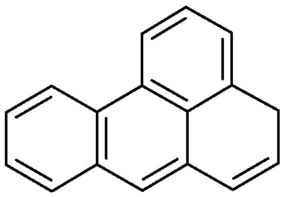 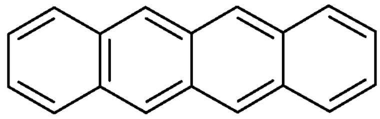

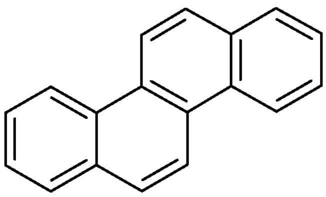 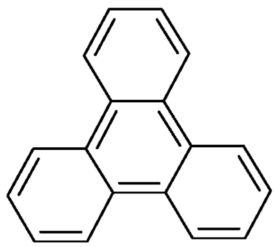

• a "flexible" part, formed from two linear or branched, saturated or unsaturated, fluorinated or nonfluorinated aliphatic chains, said chain(s) being optionally interrupted with one or more heteroatoms, with one or more metalloids and/or with one or more aromatic or nonaromatic, 4- to 6-membered (hetero)cycles, and optionally substituted with one or more groups chosen from the group constituted by hydroxyl, $-NH_2$ and oxo groups; said aliphatic chains being covalently bonded *via* a single trivalent spacer to the same carbon atom of the group Ar forming the rigid part; said spacer being an atom or a group of atoms with a valency equal to 3; and
• one or more ionic groups $-A^{x-}C^{x+}$,

$-A^{x-}$ being an anionic group covalently bonded to said rigid part, with x being an integer equal to 1 or 2, $-A^{x-}$ being chosen from the group constituted of sulfonate anions, sulfonylimide of formula $-SO_2-N^--SO_2C_yF_{2y+1}$ with y being an integer ranging from 0 to 4, borate, borane, phosphate, phosphinate, phosphonate, silicate, carbonate, sulfide, selenate, nitrate and perchlorate; and
$C^{x+}$ being a counter-cation of the anionic group - $A^x$, chosen from the group constituted of $H^+$ and alkali metal and alkaline-earth metal cations.

2. Molecule according to Claim 1, in which said polycyclic group Ar is an aromatic bicyclic group, in particular with a naphthalene aromatic backbone, and more particularly Ar is a naphthalene group.

3. Molecule according to either one of the preceding claims, in which the flexible part is formed from:
two linear or branched aliphatic chains, each of the chains containing a linear sequence of at least 6 covalent bonds.

4. Molecule according to any one of the preceding claims, in which each of the aliphatic chains is formed from a single segment or from a linear sequence of at least two chain segments, in particular from two or three chain segments of different chemical nature.

**5.** Molecule according to any one of the preceding claims, in which said flexible part is formed from two linear alkyl chains, comprising from 4 to 18 carbon atoms, optionally substituted with one or more hydroxyl groups, optionally interrupted with one or more oxygen atoms.

**6.** Molecule according to any one of the preceding claims, in which said anionic group(s) $-A^{x-}$ are sulfonate anions and/or anions of formula $-SO_2-N^--SO_2-C_yF_{2y+1}$ with y ranging from 0 to 4 and preferably equal to 1, in particular sulfonate anions.

**7.** Molecule according to any one of the preceding claims, in which $C^{x+}$ is $H^+$ or the $Li^+$ cation.

**8.** Molecule according to any one of the preceding claims, **characterized in that** it has the following structure:

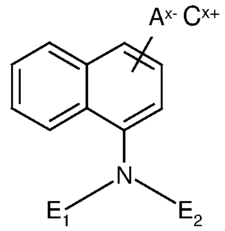

(I)

in which $A^{x-}$ and $C^{x+}$ are as defined in any one of Claims 1, 6 and 7, and $E_1$ and $E_2$ represent identical or different aliphatic chains, as defined in any one of Claims 1 and 3 to 5, in particular $E_1$ and $E_2$ represent identical or different linear alkyl chains, comprising from 6 to 16 carbon atoms, each being substituted with a hydroxyl group, optionally fluorinated, and optionally interrupted with an oxygen atom.

**9.** Use of thermotropic ionic liquid crystal molecules as defined in any one of Claims 1 to 8, in a mesomorphic state, as electrolyte in an electrochemical system.

**10.** Use according to the preceding claim, in which $C^{x+}$ represents an $Li^+$ cation, as electrolyte in a lithium battery.

**11.** Use according to Claim 10, in which $C^{x+}$ represents $H^+$, as electrolyte in a proton-exchange-membrane fuel cell, or a low-temperature electrolyzer.

**12.** Electrolyte comprising, or even being formed from, thermotropic ionic liquid crystal molecules as defined in any one of Claims 1 to 8, in a mesomorphic state, said electrolyte having in particular a viscosity of greater than or equal to 10 mPa.s at a temperature of between -60°C and 300°C.

**13.** Electrolyte according to Claim 12, **characterized in that** it has an ion conductivity at 20°C of greater than or equal to $10^{-9}$ S/cm, in particular between $10^{-7}$ S/cm and $10^{-5}$ S/cm and an ion conductivity at 200°C of greater than or equal to $10^{-5}$ S/cm.

**14.** An electrochemical system comprising an electrolyte as defined in either of Claims 12 and 13, said electrolyte preferably being impregnated on a porous separator, said electrochemical system being more particularly a battery, in particular a lithium battery.

**15.** Porous separator, **characterized in that** it is impregnated with an electrolyte as defined in either one of Claims 12 and 13.

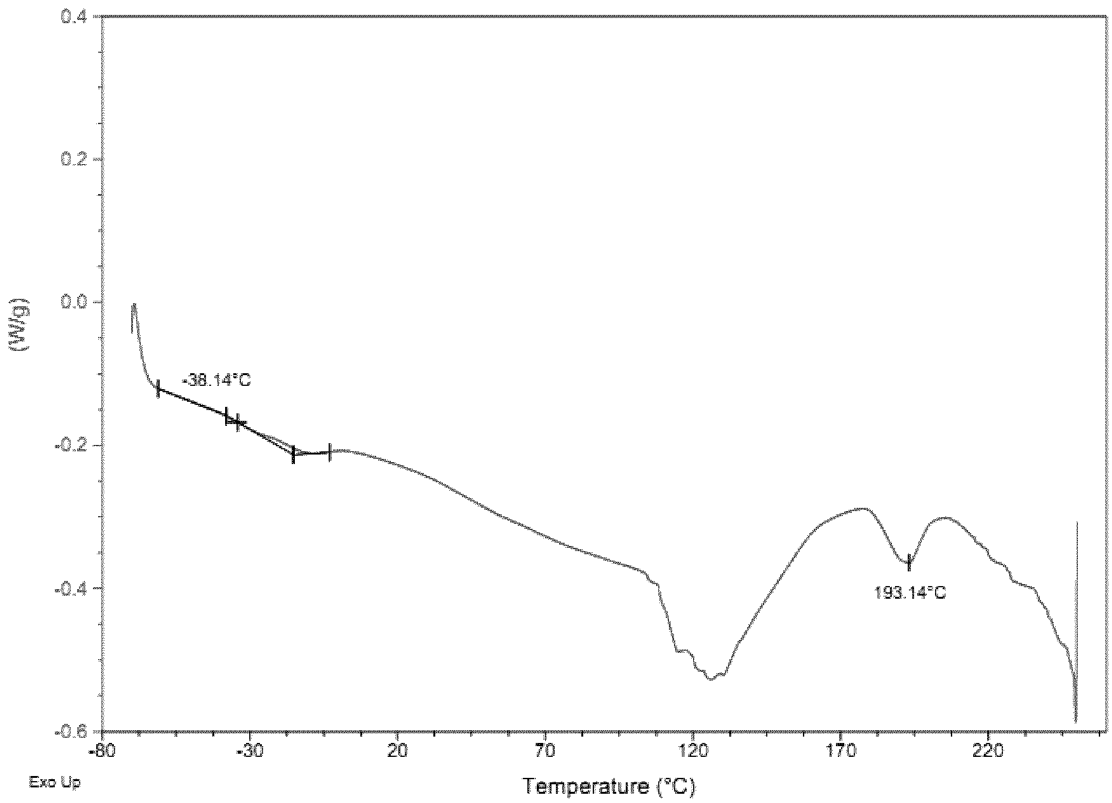


**FIGURE 1**

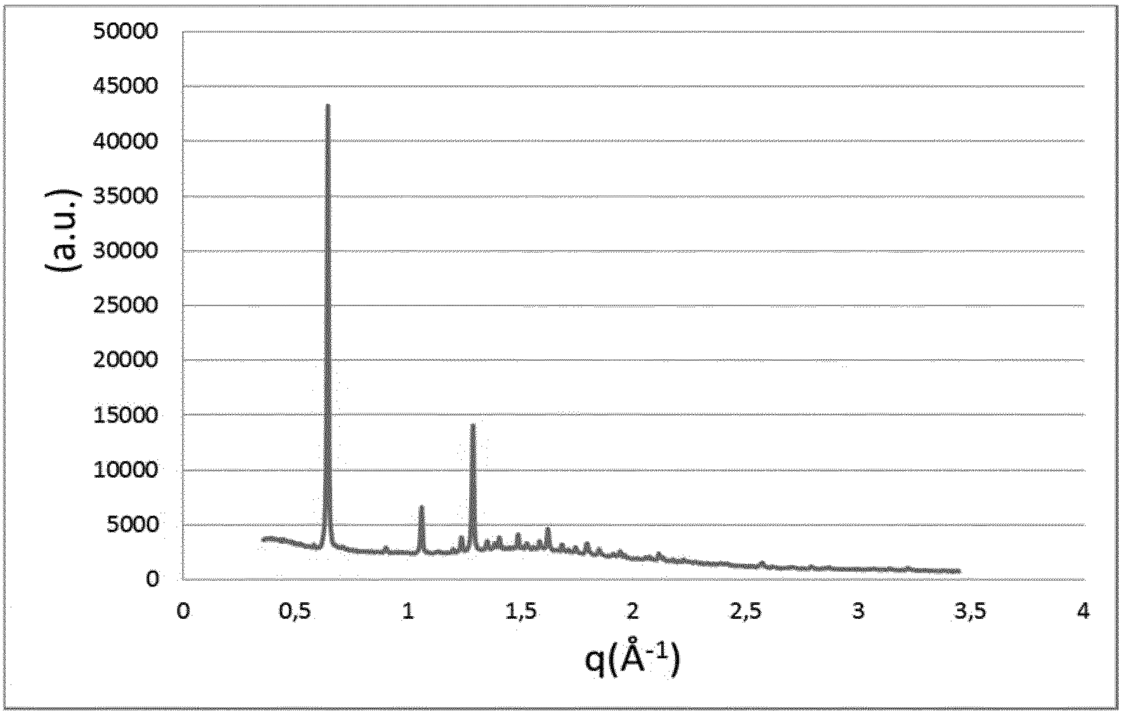


**FIGURE 2**

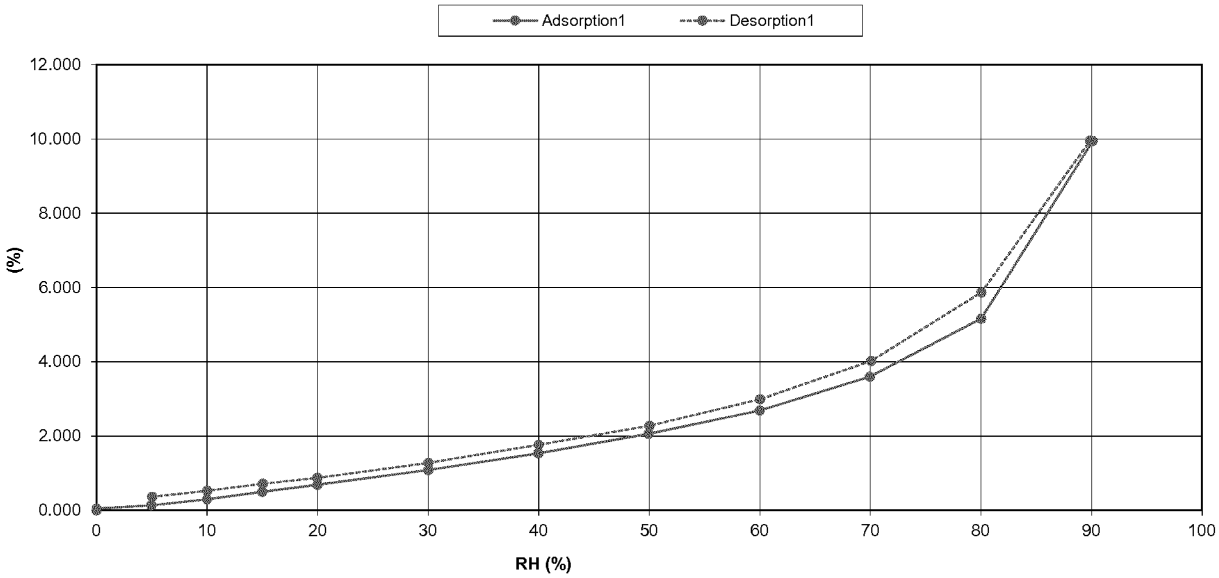


FIGURE 3

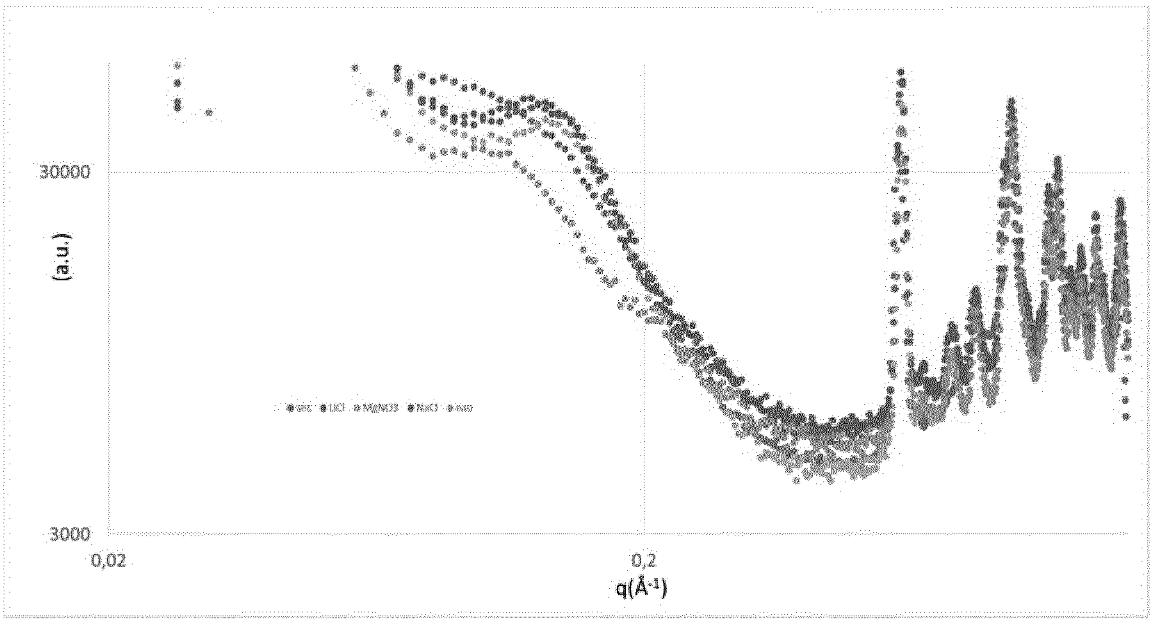


FIGURE 4

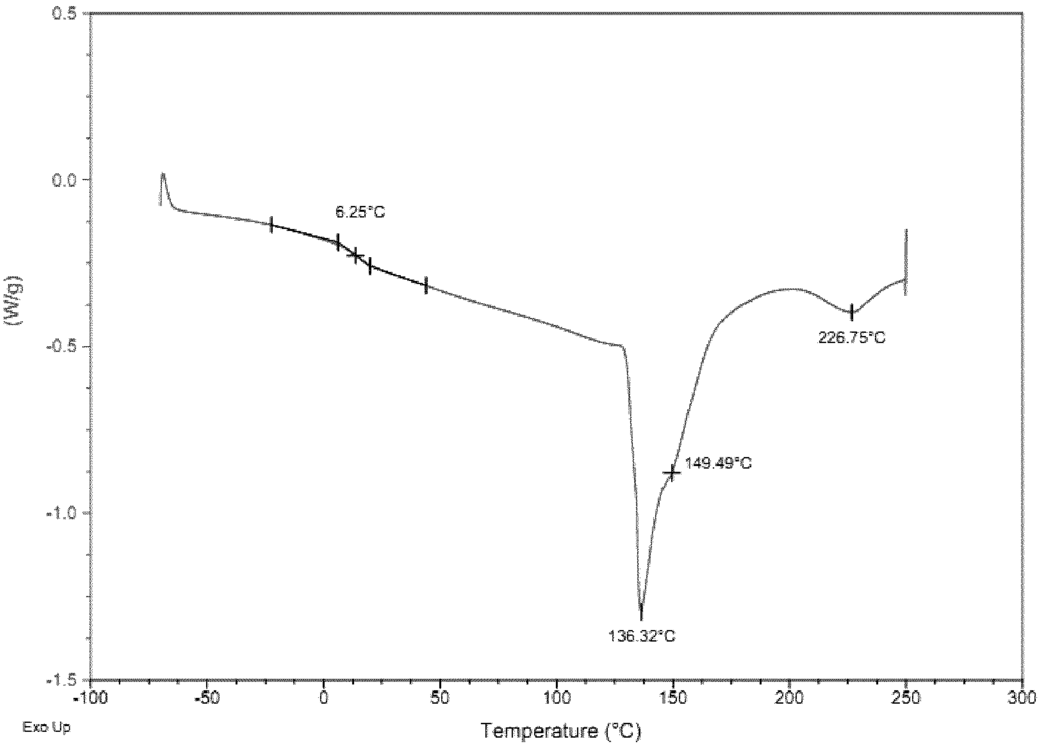


**FIGURE 5**

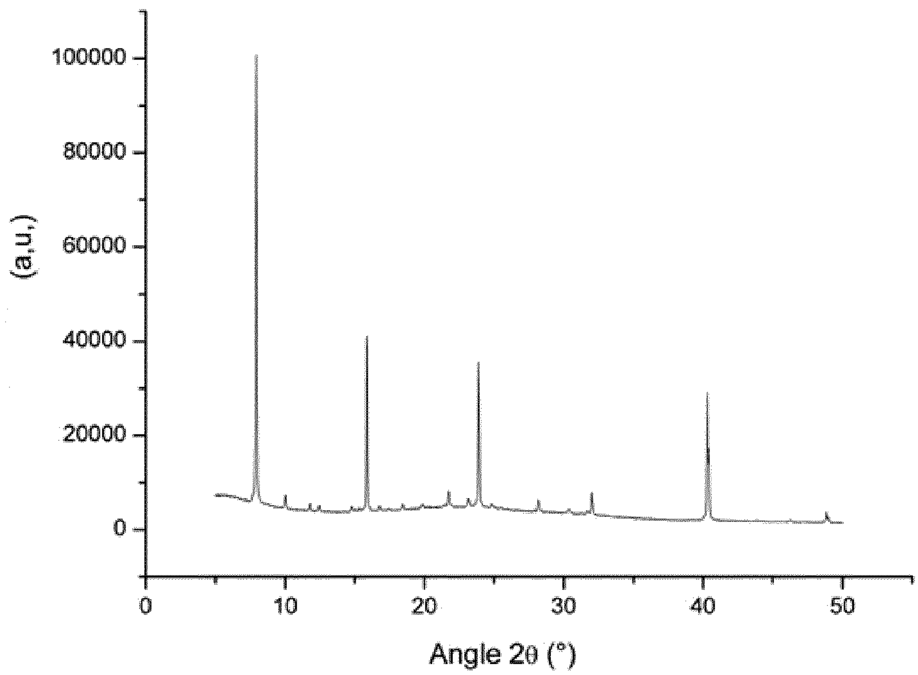


**FIGURE 6**

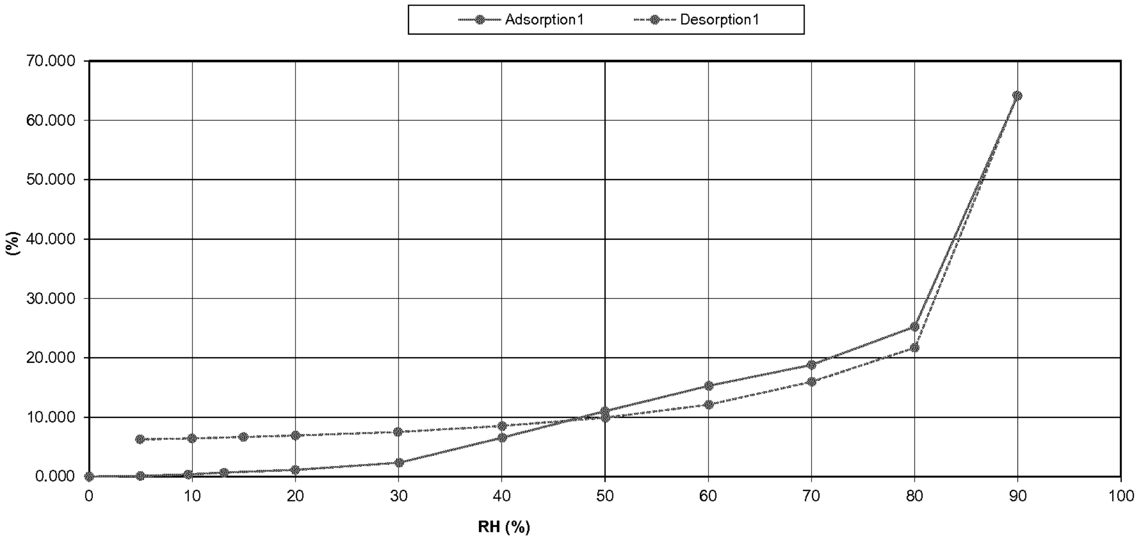


FIGURE 7

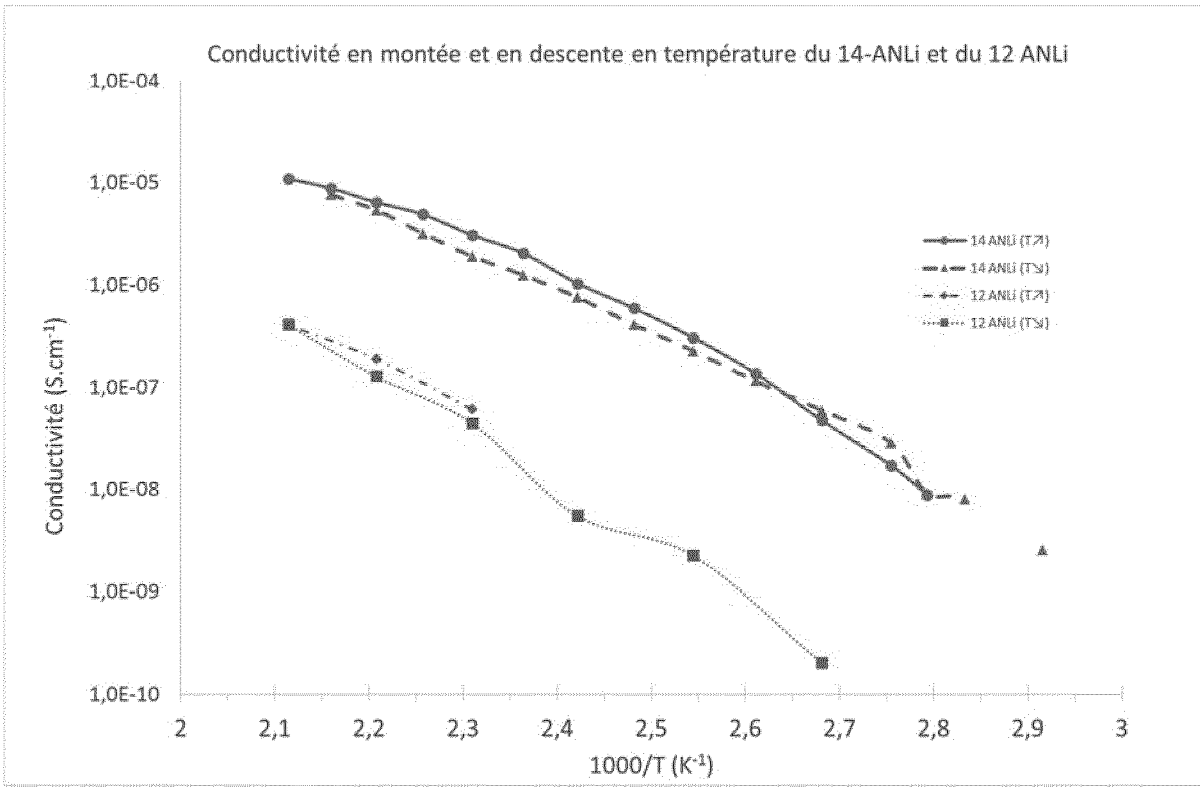


FIGURE 8

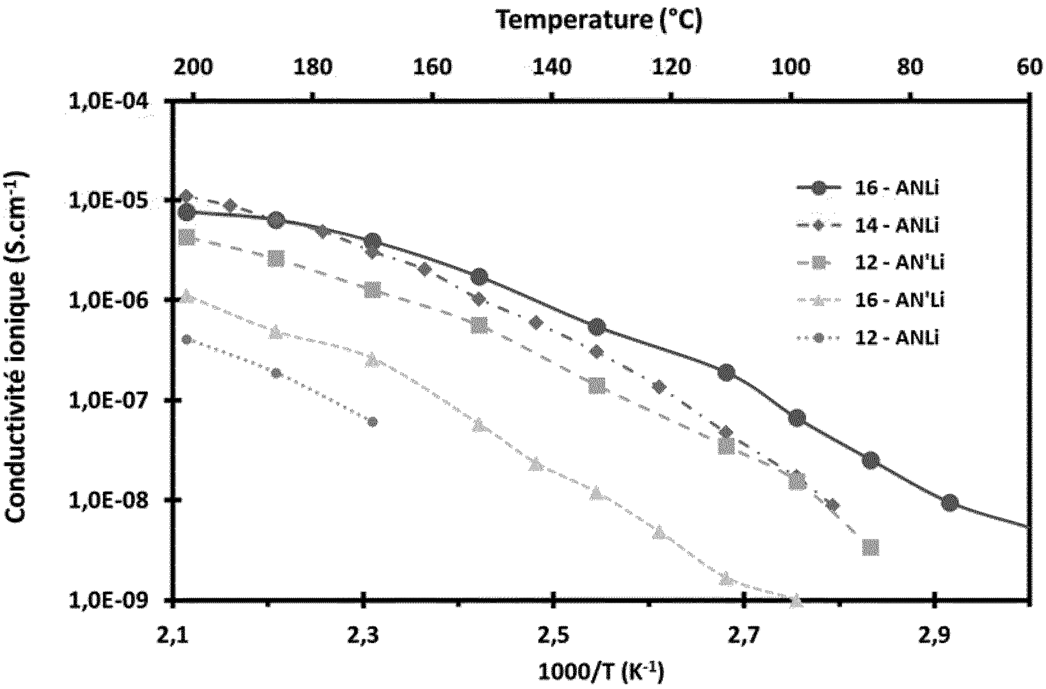
Temperature (°C)
200
180
160
140
120
100
80
60
1,0E-04
1,0E-05
1,0E-06
1,0E-07
1,0E-08
1,0E-09
Conductivité ionique (S.cm-1)
16 - ANLi
14 - ANLi
12 - AN'Li
16 - AN'Li
12 - ANLi
2,1
2,3
2,5
2,7
2,9
1000/T (K-1)

FIGURE 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0005774 A
- FR 2979630
- JP 2000336052 A
- EP 2792671 A


### Littérature non-brevet citée dans la description


- **MEZIANE et al.** *Electrochimica Acta,* 2011, vol. 57, 14-19
- **ICHIKAWA et al.** *Journal of the American Chemical Society,* vol. 134 (5), 2634-2643
- **COHEN et al.** Molecular Transport in Liquids and Glasses. *J. Chem. Phys.,* 1959, vol. 31, 1164